# EUROPEAN PATENT APPLICATION

(11) **EP 4 624 499 A1**
(43) Date of publication of application: **01.10.2025**
(21) Application number: 23905913.2
(22) Date of filing: 19.12.2023
(51) Int. Cl.: C07K 19/00, C12N 15/62, C12N 15/63, C12N 5/10, A61K 38/17, A61P 31/22

(54) **T-CELL ANTIGEN RECEPTOR, PREPARATION METHOD THEREFOR, AND USE THEREOF**

(30) Priority: 19.12.2022 CN 202211631086; 17.08.2023 CN 202311039891; 12.09.2023 CN 202311174194
(71) Applicant: Immunotech Applied Science Limited, Beijing 100176 (CN)
(72) Inventor: SUN, Jiaojun, Beijing 100176 (CN); XU, Qilong, Beijing 100176 (CN); LI, Xuejiao, Beijing 100176 (CN); LI, Xiaodan, Beijing 100176 (CN); WANG, Yu, Beijing 100176 (CN)
(74) Representative: Hautier IP - MC/EP
(86) International application number: PCT/CN2023/139719
(87) International publication number: WO 2024/131750

(57) **Abstract**

The present invention relates to the technical field of T-cell antigen receptors, and in particular to a TCR capable of recognizing and binding to a CMVpp65 antigen complex, a nucleic acid comprising a nucleotide sequence encoding the TCR, a vector containing nucleic acid molecules, a cell transducing the nucleic acid molecules or the vector, a pharmaceutical composition comprising the TCR, the nucleic acid molecules, the vector or the cell as an active component, and a use of the TCR, the nucleic acid molecules, the vector, the cell, and the pharmaceutical composition in the preparation of a drug for treating tumors or viral infections, respectively.

## Description

### TECHNICAL FIELD

The present invention relates to the field of biomedicine, and in particular to a T cell antigen receptor, a preparation method therefor and a use thereof.

### BACKGROUND ART

T cell receptor (TCR) is a molecule that enables T lymphocytes to specifically recognize antigens and initiate immune responses. It is a heterodimeric cell surface protein of the immunoglobulin superfamily, associated with invariant proteins of the CD3 complex involved in regulating signal transduction. TCR is the only receptor for specific antigenic peptides presented on the major histocompatibility complex (MHC) and is crucial for the cellular immune function of the immune system. The combination of antigen-specific TCR and MHC complex triggers direct physical contact and interaction between T cells and antigen-presenting cells, leading to a series of subsequent cellular signal transmission and other physiological reactions, thus making T cells with different antigen specificities exert immune effects on their target cells.

Cytomegalovirus (CMV) is a DNA virus belonging to the herpesvirus group and is named as cytomegalovirus, since infected cells will be swollen. CMV is one of the most common pathogens in human beings, which is widely prevalent in the population, and its infection rate in adult population can be as high as 50% to 100%. In individuals with a normal immune system, CMV infection usually does not cause any clinical symptoms, but after an asymptomatic primary infection, CMV cannot be completely eliminated, and a persistent latent infection is formed in patients. If the immune system function of individuals with CMV is suppressed, such as the onset of AIDS and immunosuppressive treatment after organ transplantation, the reactivation of latent CMV infection can lead to serious or even fatal diseases. In addition, CMV infection may be further related to the pathogenesis and progression of some tumors. For example, the CMV-pp65 antigen can be detected in half of the glioblastoma tissues. Therefore, the prevention and treatment of diseases related to CMV infection have become a direction in drug development.

At present, a solution for preparing TCR-T cells against CMV infection by using CMV-specific TCR has been developed. After TCR-T cells are reinfused into patients, patients with CMV reactivation can obtain effective immediate antiviral ability to eliminate the current reactivated virus. Moreover, patients receiving CMV-specific TCR-T reinfusion can further obtain lasting antiviral ability, and the probability of subsequent reactivation can be significantly reduced. Preliminary clinical studies also show that CMV-pp65 vaccine can prolong the progression-free survival and overall survival rate of patients with brain glioma. Therefore, CMV-pp65-targeting TCR can be further used to treat CMV-associated malignant tumors.

Both CN113881680A and CN102656188A disclose TCR that can recognize the antigen of cytomegalovirus, but the screening methods do not pay special attention to the heterogeneity of TCR recognition, and the killing effect on target cells is not obvious. CN106279404A discloses a soluble and stable heterodimeric TCR, which is a heterodimeric TCR that contains an artificial interchain disulfide bond between the α chain variable region and the β chain constant region. The TCR is soluble and stable, and can be well renatured, refolded and purified, and can specifically bind to the original ligand. However, this patent does not disclose the specific TCR for cytomegalovirus.

### SUMMARY

TCR-T therapy is effective in controlling acute CMV diseases and eliciting antiviral immune response. However, how to obtain effective T cells from either patient-derived or in vitro stimulated cytotoxic T lymphocytes and how to rapidly obtain TCR sequences thereof, to construct functional TCR-transduced specific T cells, is an obstacle to the use of TCR-T. On the basis of single-cell sequencing technology, the present invention realizes the determination of TCR pairing sequence through high-throughput sequencing, and evaluates the expression level of key genes through immune cells to conduct specific T cell receptor research.

Specifically, in one implementation of the present invention, CMV pp65 short peptide antigen is adopted to induce and amplify antigen-specific cytotoxic T lymphocytes in vitro, and TCR pairing information of positive T cells after single cell sequencing and MHC tetramer sorting is adopted to screen TCR according to the cloning sequence frequency, and to construct TCR-T. The function in vitro is verified to evaluate the function of TCR-T, so as to finally develop and obtain the specific T cell receptor of the present invention.

The HLA-A24-CMV-pp65₃₄₁₋₃₄₉ antigen complex disclosed in the present invention is composed of human leukocyte surface antigen HLA-A24 and CMV-PP65₃₄₁₋₃₄₉ short peptide (natural sequence: QYDPVAALF (SEQ ID NO: 13)), which is expressed on the surface of target cells.

In order to achieve the above objective, in the first aspect of the present invention, a TCR for recognizing human cytomegalovirus pp65 antigen is provided, wherein the TCR has the characteristic of binding to HLA-A24-CMV-pp65₃₄₁₋₃₄₉ antigen complex, and the TCR at least contains an α chain variable region and/or a β chain variable region. Preferably, the T cell antigen receptor is an αβ heterodimer, and each of them comprises a TCRα chain variable region and a TCRβ chain variable region.

In one of the embodiments, the TCRα chain variable region comprises three complementarity determining regions (CDR) of CDR1α-CDR3α, wherein the CDR3α comprises the sequence as set forth in one of SEQ ID NO: 3, SEQ ID NO: 22 and SEQ ID NO: 31, or contains an amino acid sequence with at least 85%, 90%, 95%, 98%, or at least 99% or more homology with one of SEQ ID NO: 3, SEQ ID NO: 22 and SEQ ID NO: 31. Preferably, the amino acid sequence of CDR3α in the TCRα chain variable region is as set forth in one of SEQ ID NO: 3, SEQ ID NO: 22 and SEQ ID NO: 31.

In one of the embodiments, the TCRβ chain variable region comprises three CDR regions of CDR1β-CDR3β, wherein the CDR3β contains the sequence as set forth in SEQ ID NO: 6, SEQ ID NO: 16 and SEQ ID NO: 25, or comprises an amino acid sequence with at least 85%, 90%, 95%, 98%, or at least 99% or more homology with one of SEQ ID NO: 6, SEQ ID NO: 16 and SEQ ID NO: 25. Preferably, the amino acid sequence of CDR3β in the TCRβ chain variable region is as set forth in one of SEQ ID NO: 6, SEQ ID NO: 16 and SEQ ID NO: 25.

In one of the embodiments, the CDR1α in the TCRα chain variable region comprises the amino acid sequence as set forth in SEQ ID NO: 1, or comprises an amino acid sequence with at least 85%, 90%, 95%, 98%, or at least 99% or more homology with SEQ ID NO: 1. The CDR2α comprises the amino acid sequence as set forth in SEQ ID NO: 2, or comprises the amino acid sequence with at least 85%, 90%, 95%, 98%, or at least 99% or more homology with SEQ ID NO: 2. The CDR1β in the TCRβ chain variable region comprises an amino acid sequence as set forth in one of SEQ ID NO: 4, SEQ ID NO: 14 and SEQ ID NO: 23, or comprises an amino acid sequence with at least 85%, 90%, 95%, 98%, or at least 99% or more homology with SEQ ID NO: 4, SEQ ID NO: 14 and SEQ ID NO: 23. The CDR2β comprises an amino acid sequence as set forth in one of SEQ ID NO: 5, SEQ ID NO: 15 and SEQ ID NO: 24, or comprises an amino acid sequence with at least 85%, 90%, 95%, 98%, or at least 99% or more homology with one of SEQ ID NO: 5, SEQ ID NO: 15 and SEQ ID NO: 24.

In a preferred embodiment, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: one of CARNTGKLIF (SEQ ID NO: 3), CAPSASKIIF (SEQ ID NO: 22), and CAPQFNKFYF (SEQ ID NO: 31);
CDR1β: one of SQVTM (SEQ ID NO: 4), SGHVS (SEQ ID NO: 14), and LNHDA (SEQ ID NO: 23);
CDR2β: one of ANQGSEA (SEQ ID NO: 5), FQNEAQ (SEQ ID NO: 15), and SQIVND (SEQ ID NO: 24);
CDR3β: one of CSANPTGGGTEAFF (SEQ ID NO: 6), CASSLLTRTETQYF (SEQ ID NO: 16), and CASSTTGLAGGPGNEQFF (SEQ ID NO: 25).

In a preferred embodiment, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CARNTGKLIF (SEQ ID NO: 3);
CDR1β: SQVTM (SEQ ID NO: 4);
CDR2β: ANQGSEA (SEQ ID NO: 5); and
CDR3β: CSANPTGGGTEAFF (SEQ ID NO: 6).

In a preferred embodiment, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CARNTGKLIF (SEQ ID NO: 3);
CDR1β: SGHVS (SEQ ID NO: 14);
CDR2β: FQNEAQ (SEQ ID NO: 15); and
CDR3β: CASSLLTRTETQYF (SEQ ID NO: 16).

In a preferred embodiment, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CAPSASKIIF (SEQ ID NO: 22);
CDR1β: LNHDA (SEQ ID NO: 23);
CDR2β: SQIVND (SEQ ID NO: 24); and
CDR3β: CASSTTGLAGGPGNEQFF (SEQ ID NO: 25).

In a preferred embodiment, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CAPQFNKFYF (SEQ ID NO: 31);
CDR1β: SGHVS (SEQ ID NO: 14);
CDR2β: FQNEAQ (SEQ ID NO: 15); and
CDR3β: CASSLLTRTETQYF (SEQ ID NO: 16).

In one of the embodiments, the α chain variable region of TCR of the present invention comprises an amino acid sequence of SEQ ID NO: 7, SEQ ID NO: 17, SEQ ID NO: 26 and SEQ ID NO: 32, or comprises an amino acid sequence with at least 80%, or at least 85%, or at least 90%, 92%, 94%, 96%, 98%, or 99% or more homology with SEQ ID NO: 7, SEQ ID NO: 17, SEQ ID NO: 26 and SEQ ID NO: 32.

Preferably, the α chain of TCR of the present invention comprises an amino acid sequence of SEQ ID NO: 9, SEQ ID NO: 19, SEQ ID NO: 28, and SEQ ID NO: 34, or comprises an amino acid sequence with at least 80%, or at least 85%, or at least 90%, 92%, 94%, 96%, 98%, or 99% or more homology with SEQ ID NO: 9, SEQ ID NO: 19, SEQ ID NO: 28, and SEQ ID NO: 34.

In one of the embodiments, the β chain variable region of TCR of the present invention comprises the amino acid sequence of SEQ ID NO: 8, SEQ ID NO: 18, SEQ ID NO: 27, and SEQ ID NO: 33, or comprises an amino acid sequence with at least 80%, or at least 85%, or at least 90%, 92%, 94%, 96%, 98%, or 99% or more homology with SEQ ID NO: 8, SEQ ID NO: 18, SEQ ID NO: 27, and SEQ ID NO: 33.

Preferably, the β chain of TCR of the present invention comprises the amino acid sequence of SEQ ID NO: 10, SEQ ID NO: 20, SEQ ID NO: 29, and SEQ ID NO: 35, or comprises an amino acid sequence with at least 80%, or at least 85%, or at least 90%, 92%, 94%, 96%, 98%, or 99% or more homology with SEQ ID NO: 10, SEQ ID NO: 20, SEQ ID NO: 29, and SEQ ID NO: 35.

In a specific embodiment of the present invention, preferably, the amino acids of the α chain and the β chain of the TCR of the present invention are directly or indirectly connected, preferably indirectly connected, and more preferably connected by using 2A peptides. The 2A peptides are short peptides (18-25 amino acids) derived from viruses and often referred to as a "self-cleavage" peptide, which enables a single transcription product to produce multiple proteins. The 2A peptides adopted in the present invention include but are not limited to P2A, T2A, E2A and F2A.

In some embodiments of the present invention, the 2A peptide, such as P2A, can further be linked with a furin cleavage site, a ser-gly linker (sgsg) and a p2a ribosomal skipping peptide, so as to form an fp2A (furin-SGSG-p2A) linking structure.

Further preferably, the fp2A contains the amino acid sequence as set forth in SEQ ID NO: 11.

In a specific embodiment of the present invention, the linkage order of α chain and β chain can be α chain, fp2A and β chain, or the linkage order can be β chain, fp2A and α chain.

In a specific embodiment of the present invention, the TCR of the present invention contains the amino acid sequences as set forth in SEQ ID NO: 12, SEQ ID NO: 21, SEQ ID NO: 30, and SEQ ID NO: 36, or contains an amino acid sequence with at least 80%, or at least 85%, or at least 90%, 92%, 94%, 96%, 98%, or 99% or more homology with SEQ ID NO: 12, SEQ ID NO: 21, SEQ ID NO: 30, and SEQ ID NO: 36.

The CMV pp65₄₉₃₋₅₀₃ short peptide according to the present invention (natural sequence: NLVPMVATV (SEQ ID NO:46)) expresses on the surface of target cells.

In order to achieve the above objective, a TCR for recognizing human cytomegalovirus pp65 antigen is provided, wherein the TCR has the characteristic of binding to CMV pp65₄₉₃₋₅₀₃ antigen complex, and the TCR at least comprises an α chain variable region and/or a β chain variable region. Preferably, the T cell antigen receptor is an αβ heterodimer, and each of them comprises a TCRα chain variable region and a TCRβ chain variable region.

In one of the embodiments, the TCRα chain variable region contains three complementarity determining regions (CDR) of CDR1α-CDR3α, wherein the CDR3α comprises the sequence as set forth in one of SEQ ID NO: 37, SEQ ID NO: 49, SEQ ID NO: 60 and SEQ ID NO: 69, or comprises an amino acid sequence with at least 85%, 90%, 95%, 98%, or at least 99% or more homology with one of SEQ ID NO: 37, SEQ ID NO: 49, SEQ ID NO: 60 and SEQ ID NO: 69. Preferably, the amino acid sequence of CDR3α in the TCRα chain variable region is as set forth in one of SEQ ID NO: 37, SEQ ID NO: 49, SEQ ID NO: 60 and SEQ ID NO: 69.

In one of the embodiments, the TCRβ chain variable region comprises three CDR regions of CDR1β-CDR3β, wherein the CDR3β contains the sequence as set forth in SEQ ID NO: 40, SEQ ID NO: 52, SEQ ID NO: 63 and SEQ ID NO: 72, or comprises an amino acid sequence with at least 85%, 90%, 95%, 98%, or at least 99% or more homology with one of SEQ ID NO: 40, SEQ ID NO: 52, SEQ ID NO: 63 and SEQ ID NO: 72. Preferably, the amino acid sequence of CDR3β in the TCRβ chain variable region is as set forth in one of SEQ ID NO: 40, SEQ ID NO: 52, SEQ ID NO: 63 and SEQ ID NO: 72.

In one of the embodiments, the CDR1α in the TCRα chain variable region comprises the amino acid sequence as set forth in one of SEQ ID NO: 1, SEQ ID NO: 47 and SEQ ID NO: 58, or comprises an amino acid sequence with at least 85%, 90%, 95%, 98%, or at least 99% or more homology with SEQ ID NO: 1, SEQ ID NO: 47 and SEQ ID NO: 58. The CDR2α comprises the amino acid sequence as set forth in one of SEQ ID NO: 2, SEQ ID NO: 48 and SEQ ID NO: 59, or comprises the amino acid sequence with at least 85%, 90%, 95%, 98%, or at least 99% or more homology with SEQ ID NO: 2, SEQ ID NO: 48 and SEQ ID NO: 59.

The CDR1β in the TCRβ chain variable region contains an amino acid sequence as set forth in one of SEQ ID NO: 38, SEQ ID NO: 50, SEQ ID NO: 61 and SEQ ID NO: 70, or comprises an amino acid sequence with at least 85%, 90%, 95%, 98%, or at least 99% or more homology with one of SEQ ID NO: 38, SEQ ID NO: 50, SEQ ID NO: 61 and SEQ ID NO: 70. The CDR2β comprises an amino acid sequence as set forth in one of SEQ ID NO: 39, SEQ ID NO: 51, SEQ ID NO: 62 and SEQ ID NO: 71, or comprises an amino acid sequence with at least 85%, 90%, 95%, 98%, or at least 99% or more homology with one of SEQ ID NO: 39, SEQ ID NO: 51, SEQ ID NO: 62 and SEQ ID NO: 71.

In a preferred embodiment, the amino acid sequences of CDR1α-CDR3α of the α chain and CDR1β-CDR3β of the β chain of the TCR are shown below:
CDR1α: selected from one of SSNFYA (SEQ ID NO: 1), DSSSTY (SEQ ID NO: 47), and TSGFNG (SEQ ID NO: 58);
CDR2α: selected from one of MTLNGDE (SEQ ID NO: 2), IFSNMDM (SEQ ID NO: 48), and NVLDGL (SEQ ID NO: 59);
CDR3α: selected from one of CASINFNKFYF (SEQ ID NO: 37), CAEFTGTASKLTF (SEQ ID NO: 49), CAVTYNNARLMF (SEQ ID NO: 60), and CARNYGQNFVF (SEQ ID NO: 69);
CDR1β: selected from one of MDHEN (SEQ ID NO: 38), GTSNPN (SEQ ID NO: 50), MNHEY (SEQ ID NO: 61), and DFQATT (SEQ ID NO: 70);
CDR2β: selected from one of SYDVKM (SEQ ID NO: 39), SVGIG (SEQ ID NO: 51), SMNVEV (SEQ ID NO: 62), and SNEGSKA (SEQ ID NO: 71); and
CDR3β: selected from one of CASSPLNGGATEAFF (SEQ ID NO: 40), CAWSDRAAFTDTQYF (SEQ ID NO: 52), CASSSVAGGRIEQFF (SEQ ID NO: 63), CSARDIKAQQWNIQYF (SEQ ID NO: 72).

In a preferred embodiment, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CASINFNKFYF (SEQ ID NO: 37);
CDR1β: MDHEN (SEQ ID NO: 38);
CDR2β: SYDVKM (SEQ ID NO: 39); and
CDR3β: CASSPLNGGATEAFF (SEQ ID NO: 40).

In a preferred embodiment, the amino acid sequences of CDR1α-CDR3α of the α chain and CDR1β-CDR3β of the β chain of the TCR are shown below:
CDR1α: DSSSTY (SEQ ID NO: 47);
CDR2α: IFSNMDM (SEQ ID NO: 48);
CDR3α: CAEFTGTASKLTF (SEQ ID NO: 49);
CDR1β: GTSNPN (SEQ ID NO: 50);
CDR2β: SVGIG (SEQ ID NO: 51); and
CDR3β: CAWSDRAAFTDTQYF (SEQ ID NO: 52).

In a preferred embodiment, the amino acid sequences of CDR1α-CDR3α of the α chain and CDR1β-CDR3β of the β chain of the TCR are shown below:
CDR1α: TSGFNG (SEQ ID NO: 58);
CDR2α: NVLDGL (SEQ ID NO: 59);
CDR3α: CAVTYNNARLMF (SEQ ID NO: 60);
CDR1β: MNHEY (SEQ ID NO: 61);
CDR2β: SMNVEV (SEQ ID NO: 62); and
CDR3β: CASSSVAGGRIEQFF (SEQ ID NO: 63).

In a preferred embodiment, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CARNYGQNFVF (SEQ ID NO: 69);
CDR1β: DFQATT (SEQ ID NO: 70);
CDR2β: SNEGSKA (SEQ ID NO: 71); and
CDR3β: CSARDIKAQQWNIQYF (SEQ ID NO: 72).

In one of the embodiments, the α chain variable region of TCR of the present invention comprises an amino acid sequence of SEQ ID NO: 41, SEQ ID NO: 53, SEQ ID NO: 64 and SEQ ID NO: 73, or comprises an amino acid sequence with at least 80%, or at least 85%, or at least 90%, 92%, 94%, 96%, 98%, or 99% or more homology with SEQ ID NO: 41, SEQ ID NO: 53, SEQ ID NO: 64 and SEQ ID NO: 73.

Preferably, the α chain of TCR of the present invention comprises an amino acid sequence of SEQ ID NO: 43, SEQ ID NO: 55, SEQ ID NO: 66, and SEQ ID NO: 75, or comprises an amino acid sequence having at least 80%, or at least 85%, or at least 90%, 92%, 94%, 96%, 98%, or 99% or more homology with SEQ ID NO: 43, SEQ ID NO: 55, SEQ ID NO: 66, and SEQ ID NO: 75.

In one of the embodiments, the β chain variable region of TCR of the present invention comprises the amino acid sequence of SEQ ID NO: 42, SEQ ID NO: 54, SEQ ID NO: 65, and SEQ ID NO: 74, or comprises an amino acid sequence with at least 80%, or at least 85%, or at least 90%, 92%, 94%, 96%, 98%, or 99% or more homology with SEQ ID NO: 42, SEQ ID NO: 54, SEQ ID NO: 65, and SEQ ID NO: 74.

Preferably, the β chain of TCR of the present invention comprises the amino acid sequence of SEQ ID NO: 44, SEQ ID NO: 56, SEQ ID NO: 67, and SEQ ID NO: 76, or comprises an amino acid sequence having at least 80%, or at least 85%, or at least 90%, 92%, 94%, 96%, 98%, or 99% or more homology with SEQ ID NO: 44, SEQ ID NO: 56, SEQ ID NO: 67, and SEQ ID NO: 76.

In a specific embodiment of the present invention, preferably, the amino acids of the α chain and the β chain of the TCR of the present invention are directly or indirectly connected, preferably indirectly connected, and more preferably connected by using 2A peptides. The 2A peptides are short peptides (18-25 amino acids) derived from viruses and often referred to as a "self-cleavage" peptide, which enables a single transcription product to produce multiple proteins. The 2A peptides adopted in the present invention include but are not limited to P2A, T2A, E2A and F2A.

In some embodiments of the present invention, the 2A peptide, such as P2A, can further be linked with a furin cleavage site, a ser-gly linker (sgsg) and a p2a ribosomal skipping peptide, so as to form an fp2A(furin-SGSG-p2A) linking structure.

Further preferably, the fp2Acomprises the amino acid sequence as set forth in SEQ ID NO: 11.

In a specific embodiment of the present invention, the linkage order of α chain and β chain can be α chain, fp2A and β chain, or the linkage order can be β chain, fp2A and α chain.

In a specific embodiment of the present invention, the TCR of the present invention comprises the amino acid sequences as set forth in SEQ ID NO: 45, SEQ ID NO: 57, SEQ ID NO: 68, and SEQ ID NO: 77, or comprises an amino acid sequence having at least 80%, or at least 85%, or at least 90%, 92%, 94%, 96%, 98%, or 99% homology to SEQ ID NO: 45, SEQ ID NO: 57, SEQ ID NO: 68,or SEQ ID NO: 77.

The CMV pp65₅₀₁₋₅₀₉ short peptide (ATVQGQNLK (SEQ ID NO: 89)) is expressed on the surface of target cells.

In order to achieve the above objective, a TCR for recognizing human cytomegalovirus pp65 antigen is provided, wherein the TCR has the characteristic of binding to CMV pp65₅₀₁₋₅₀₉ antigen complex, and the TCR at least comprises an α chain variable region and/or a β chain variable region. Preferably, the T cell antigen receptor is an αβ heterodimer, and each of them comprises a TCRα chain variable region and a TCRβ chain variable region.

In one of the embodiments, the TCRα chain variable region comprises three complementarity determining regions (CDR) of CDR1α-CDR3α, wherein the CDR3α contains the sequence as set forth in one of CVITTSGTYKYIF (SEQ ID NO: 80), CAYRSFYTGANSKLTF (SEQ ID NO: 92), and CVVHSGGSYIPTF (SEQ ID NO: 103), or comprises an amino acid sequence with at least 85%, 90%, 95%, 98%, or at least 99% homology to one of SEQ ID NO: 80, SEQ ID NO: 92 and SEQ ID NO: 103.

In one of the embodiments, the TCRβ chain variable region comprises three CDR regions of CDR1β-CDR3β, wherein the CDR3β comprises the sequence as set forth in one of CASTINTYEQYF (SEQ ID NO: 83), CASSLYGGPGDQPQHF (SEQ ID NO: 95), and CASAQTIGAYNEQFF (SEQ ID NO: 106), or comprises an amino acid sequence with at least 85%, 90%, 95%, 98%, or at least 99% homology to one of SEQ ID NO: 83, SEQ ID NO: 95 and SEQ ID NO: 106.

In one of the embodiments, the CDR1α in the TCRα chain variable region comprises the amino acid sequence as set forth in one of SEQ ID NO: 78, SEQ ID NO: 90, and SEQ ID NO: 101, or comprises an amino acid sequence with at least 85%, 90%, 95%, 98%, or at least 99% homology to one of SEQ ID NO: 78, SEQ ID NO: 90, and SEQ ID NO: 101. The CDR2α contains the amino acid sequence as set forth in one of SEQ ID NO: 79, SEQ ID NO: 91, and SEQ ID NO: 102, or comprises the amino acid sequence with at least 85%, 90%, 95%, 98%, or at least 99% or more homology with one of SEQ ID NO: 79, SEQ ID NO: 91, and SEQ ID NO: 102.

The CDR1β in the TCRβ chain variable region comprises an amino acid sequence as set forth in one of SEQ ID NO: 81, SEQ ID NO: 93 and SEQ ID NO: 104, or comprises an amino acid sequence with at least 85%, 90%, 95%, 98%, or at least 99% homology to SEQ ID NO: 81, SEQ ID NO: 93 and SEQ ID NO: 104. The CDR2β comprises an amino acid sequence as set forth in one of SEQ ID NO: 82, SEQ ID NO: 94 and SEQ ID NO: 105, or comprises an amino acid sequence with at least 85%, 90%, 95%, 98%, or at least 99% homology to one of SEQ ID NO: 82, SEQ ID NO: 94 and SEQ ID NO: 105.
CDR1α: selected from one of VSGLRG (SEQ ID NO: 78), TSESDYY (SEQ ID NO: 90), and NSASQS (SEQ ID NO: 101);
CDR2α: selected from one of LYSAGEE (SEQ ID NO: 79), QEAYKQQN (SEQ ID NO: 91), and VYSSGN (SEQ ID NO: 102);
CDR3α: selected from one of CVITTSGTYKYIF (SEQ ID NO: 80), CAYRSFYTGANSKLTF (SEQ ID NO: 92), and CVVHSGGSYIPTF (SEQ ID NO: 103);
CDR1β: selected from one of MNHNS (SEQ ID NO: 81), SGHDT (SEQ ID NO: 93), and MNHNY (SEQ ID NO: 104);
CDR2β: selected from one of SASEGT (SEQ ID NO: 82), YYEEEE (SEQ ID NO: 94), and SVGAGI (SEQ ID NO: 105); and
CDR3β: selected from one of CASTINTYEQYF (SEQ ID NO: 83), CASSLYGGPGDQPQHF (SEQ ID NO: 95), and CASAQTIGAYNEQFF (SEQ ID NO: 106).

In a preferred embodiment, the amino acid sequences of CDR1α-CDR3α of the α chain and CDR1β-CDR3β of the β chain of the TCR are shown below:
CDR1α: VSGLRG (SEQ ID NO: 78);
CDR2α: LYSAGEE (SEQ ID NO: 79);
CDR3α: CVITTSGTYKYIF (SEQ ID NO: 80);
CDRlβ: MNHNS (SEQ ID NO: 81);
CDR2β: SASEGT (SEQ ID NO: 82); and
CDR3β: CASTINTYEQYF (SEQ ID NO: 83).

In a preferred embodiment, the amino acid sequences of CDR1α-CDR3α of the α chain and CDR1β-CDR3β of the β chain of the TCR are shown below:
CDRlα: TSESDYY (SEQ ID NO: 90);
CDR2α: QEAYKQQN (SEQ ID NO: 91);
CDR3α: CAYRSFYTGANSKLTF (SEQ ID NO: 92);
CDR1β: SGHDT (SEQ ID NO: 93);
CDR2β: YYEEEE (SEQ ID NO: 94); and
CDR3β: CASSLYGGPGDQPQHF (SEQ ID NO: 95).

In a preferred embodiment, the amino acid sequences of CDR1α-CDR3α of the α chain and CDR1β-CDR3β of the β chain of the TCR are shown below:
CDRlα: NSASQS (SEQ ID NO: 101);
CDR2α: VYSSGN (SEQ ID NO: 102);
CDR3α: CVVHSGGSYIPTF (SEQ ID NO: 103);
CDR1β: MNHNY (SEQ ID NO: 104);
CDR2β: SVGAGI (SEQ ID NO: 105); and
CDR3β: CASAQTIGAYNEQFF (SEQ ID NO: 106).

In one of the embodiments, the α chain variable region of TCR of the present invention comprises an amino acid sequence in one of SEQ ID NO: 84, SEQ ID NO: 96, and SEQ ID NO: 107, or comprises an amino acid sequence with at least 80%, or at least 85%, or at least 90%, 92%, 94%, 96%, 98%, or 99% or more homology with one of SEQ ID NO: 84, SEQ ID NO: 96, and SEQ ID NO: 107.

Preferably, the α chain of TCR of the present invention comprises an amino acid sequence in one of SEQ ID NO: 86, SEQ ID NO: 98, and SEQ ID NO: 109, or comprises an amino acid sequence with at least 80%, or at least 85%, or at least 90%, 92%, 94%, 96%, 98%, or 99% homology to one of SEQ ID NO: 86, SEQ ID NO: 98, and SEQ ID NO: 109.

In one of the embodiments, the β chain variable region of TCR of the present invention contains the amino acid sequence in one of SEQ ID NO: 85, SEQ ID NO: 97, and SEQ ID NO: 108, or comprises an amino acid sequence with at least 80%, or at least 85%, or at least 90%, 92%, 94%, 96%, 98%, or 99% homology to one of SEQ ID NO: 85, SEQ ID NO: 97, and SEQ ID NO: 108.

Preferably, the β chain of TCR of the present invention comprises the amino acid sequence in one of SEQ ID NO: 87, SEQ ID NO: 99, and SEQ ID NO: 110, or comprises an amino acid sequence with at least 80%, or at least 85%, or at least 90%, 92%, 94%, 96%, 98%, or 99% homology to one of SEQ ID NO: 87, SEQ ID NO: 99, and SEQ ID NO: 110.

In a specific embodiment of the present invention, preferably, the amino acids of the α chain and the β chain of the TCR of the present invention are directly or indirectly connected, preferably indirectly connected, and more preferably connected by using 2A peptides. The 2A peptides are short peptides (18-25 amino acids) derived from viruses and often referred to as a "self-cleavage" peptide, which enables a single transcription product to produce multiple proteins. The 2A peptides adopted in the present invention include but are not limited to P2A, T2A, E2A and F2A.

In some embodiments of the present invention, the 2A peptide, such as P2A, can further be linked with a furin cleavage site, a ser-gly linker (sgsg) and a p2a ribosomal skipping peptide, so as to form an fp2A(furin-SGSG-p2A) linking structure.

Further preferably, the fp2A comprises the amino acid sequence as set forth in SEQ ID NO: 11.

In a specific embodiment of the present invention, the linkage order of α chain and β chain can be α chain, fp2A and β chain, or the linkage order can be β chain, fp2A and α chain.

In a specific embodiment of the present invention, the TCR of the present invention comprises the amino acid sequences as set forth in one of SEQ ID NO: 88, SEQ ID NO: 100, and SEQ ID NO: 111, or comprises an amino acid sequence with at least 80%, or at least 85%, or at least 90%, 92%, 94%, 96%, 98%, or 99% homology to one of SEQ ID NO: 88, SEQ ID NO: 100, and SEQ ID NO: 111.

In the second aspect of the present invention, a nucleic acid is provided, wherein the nucleic acid contains a nucleotide sequence encoding the TCR of the first aspect of the present invention or a complementary sequence thereof.

In the technical solution of the present invention, the nucleic acid sequence can be single-stranded or double-stranded, and can be either DNA or RNA.

In the preferred technical solution of the present invention, the nucleic acid sequence can be codon-optimized. Further preferably, the codon optimization includes replacing a large number of rare codons used by viruses with the corresponding mammalian codons and/or removing the instability motifs and/or the hidden splicing sites of mRNA.

In the third aspect of the present invention, an expression vector is provided, wherein the expression vector contains the nucleic acid according to any one of the present inventions.

In the preferred technical solution of the present invention, the expression vector can be expressed under in vivo, in vitro, or ex vivo conditions. Further preferably, the expression vector is continuously expressed at a high level in the in-vivo cells.

In the preferred technical solution of the present invention, the expression vector can be a prokaryotic expression vector or a retroviral vector.

In the preferred technical solution of the present invention, the expression vector can be Rous sarcoma virus (RSV), lentivirus, human immunodeficiency virus (HIV), murine leukemia virus (MLV), equine infectious anemia virus (EIAV), mouse mammary tumor virus (MMTV), Fujinami sarcoma virus (FuSV), FBR murine osteosarcoma virus (FBR MSV), Moloney murine leukemia virus (Mo-MLV), Moloney murine sarcoma virus (Mo-MSV), Abelson murine leukemia virus (A-MLV), avian myelocytomatosis virus 29 (MC29) or avian erythroblastosis virus (AEV), etc. More preferably, the expression vector is a lentiviral expression vector.

In the fourth aspect of the present invention, a host cell is provided, wherein the host cell contains the nucleic acid or expression vector according to any one of the present inventions.

In the preferred technical solution of the present invention, the host cell can be eukaryotic or prokaryotic. More preferably, the host cells are eukaryotic cells, including but not limited to yeast cells, 293 cells, CHO cells, etc.

In the fifth aspect of the present invention, an immune cell is provided, wherein the immune cell expresses the T cell antigen receptor according to the present invention.

In the preferred technical solution of the present invention, the immune cell includes one or more nucleic acid sequences according to any one of the present inventions.

In the preferred technical solution of the present invention, the immune cells include but are not limited to stem cells and lymphocytes (including T cells and B cells). Further, the immune cell is a B cell, wherein the B cell expresses the above-mentioned antibody or antigen-binding fragment thereof. The immune cell is a T cell, wherein the antigen receptor structure of the T cell is defined as above.

In the preferred technical solution of the present invention, the T cell can be CD4⁺T, CD8⁺T, etc.

In the preferred technical solution of the present invention, the immune cell is isolated from T cells of a subject.

In the preferred technical solution of the present invention, the immune cell is T cell from a CMV serum-negative donor.

In the sixth aspect of the present invention, a preparation method for immune cells is provided, wherein the method includes transducing the nucleic acid sequence encoding the above-mentioned T cell antigen receptor into the immune cells for expression.

In the preferred technical solution of the present invention, the immune cells include but are not limited to stem cells and lymphocytes (including T cells and B cells). Further, the immune cell is a B cell, wherein the B cell expresses the above-mentioned antibody or antigen-binding fragment thereof. The immune cell is a T cell, wherein the antigen receptor structure of the T cell is defined as above.

In the preferred technical solution of the present invention, the step of knocking out the endogenous TCR of the cell is further provided. Specifically, the guide for targeting endogenous TCR can be constructed into a lentiviral vector and co-transferred to T cells with a packaging plasmid and a transfection reagent.

In the seventh aspect of the present invention, a preparation method for recombinant T cells is provided, wherein the method includes the following steps:
1) obtaining the nucleic acid described in any one of the second aspects of the present invention by cloning from positive T cells;
2) isolating and culturing the primary T cells; and
3) delivering the nucleic acid obtained in step 1) to the primary T cell described in step 2), so as to obtain a recombinant T cell expressing the T cell antigen receptors described in any one of the present inventions.

In the preferred technical solution of the present invention, the T cells are selected from hematopoietic stem cell-derived or peripheral blood lymphocyte (PBL)-derived T cells.

In the eighth aspect of the present invention, a preparation method for T cell antigen receptor is provided, wherein the method includes the following steps:
(1) obtaining the nucleic acid described in any one of the second aspects of the present invention by cloning from positive T cells;
(2) linking the nucleic acid obtained in step (1) to a vector skeleton to obtain an expression vector;
(3) transforming the expression vector obtained in step (2) into a host cell, and then inducing its expression; and
(4) obtaining antibodies or antigen-binding fragments thereof, or T cell antigen receptors.

**In** the preferred technical solution of the present invention, the positive T cells are specifically bound to cytomegalovirus (CMV) phosphoprotein pp65 antigenic peptide presented by MHC. Further preferably, the MHC-presented cytomegalovirus (CMV) phosphoprotein pp65 antigenic peptide complexes are monomeric or multimeric complexes.

**In** the ninth aspect of the present invention, use of the T cell antigen receptor described in any one of the first aspects, the nucleic acid described in any one of the second aspects, the expression vector described in the third aspect, the host cell described in the fourth aspect, and the immune cell described in the fifth aspect in preparing products for diagnosing or treating tumors or CMV-related diseases is provided.

**In** the preferred technical solution of the present invention, the CMV-related diseases are selected from neonatal CMV inclusion body disease, acute acquired CMV infection or diseases caused by CMV infection in immunocompromised individuals.

**In** the preferred technical solutions of the present invention, the described CMV-related diseases are selected from hepatic, splenic, or central nervous system disorders, disabilities, infectious mononucleosis, skeletal muscle pain, CMV retinitis, gastrointestinal CMV or encephalitis, etc.

**In** the tenth aspect of the present invention, use of the T cell antigen receptor described in any one of the present inventions, the nucleic acid described in any one of the present inventions, the expression vector described in the present invention, the host cell described in the present invention, the immune cell described in the present invention in T cell labeling, detection, cell sorting or activation is provided.

**In** the eleventh aspect of the present invention, a pharmaceutical composition is provided, wherein the pharmaceutical composition includes any one of the following groups:
1) the T cell antigen receptor described in the present invention;
2) the nucleic acid described in the present invention;
3) the expression vector described in the present invention;
4) the host cell described in the present invention; and
5) the immune cells described in the present invention.

In the preferred technical solution of the present invention, the pharmaceutical composition can further contain pharmaceutically acceptable excipients.

In the preferred technical solution of the present invention, the pharmaceutical composition can further be used together with other therapeutic agents. Further preferably, the therapeutic agent can be an immunomodulator.

In the twelfth aspect of the present invention, a kit is provided, wherein the kit includes any one of the following groups:
1) the T cell antigen receptor described in any one of the present inventions;
2) the nucleic acid described in any one of the present inventions;
3) the expression vector described in any one of the present inventions;
4) the host cell described in any one of the present inventions; and
5) the immune cells described in any one of the present inventions.

In the thirteenth aspect of the present invention, a method for detecting cytomegalovirus (CMV) phosphoprotein pp65 is provided, wherein the method includes contacting a sample to be tested with the T cell antigen receptor described in the present invention, and then detecting a complex formed by cytomegalovirus (CMV) phosphoprotein pp65 and the T cell antigen receptor.

Preferably, the detection of cytomegalovirus (CMV) phosphoprotein pp65 refers to detecting the presence or content of cytomegalovirus (CMV) phosphoprotein pp65, wherein the existence indicates the presence or absence, and the content can be the expression quantity or protein concentration.

Preferably, the antibody or antigen-binding fragment thereof, or the T cell antigen receptor, includes a detectable marker.

In a specific embodiment of the present invention, the marker can be His and/or HA.

In one of the implementations, the method for detecting cytomegalovirus (CMV) phosphoprotein pp65 is not a method for diagnosing diseases. Firstly, the sample to be tested is not an organism or an isolated tissue or cell thereof, and secondly, even if the cytomegalovirus (CMV) phosphoprotein pp65 exists in the organism, or the organism contains a certain concentration or expression level of the cytomegalovirus (CMV) phosphoprotein pp65, a disease cannot be determined, but it is only a possibility.

In the fourteenth aspect of the present invention, a method for treating and/or preventing CMV-related diseases is provided, wherein the method includes administering to an individual an effective amount of the T cell antigen receptor, the nucleic acid, the expression vector, the host cell, the immune cell or the pharmaceutical composition described in the present invention.

Preferably, the method includes the step of adoptively transferring T cells expressing the T cell antigen receptor described in the present invention to a subject.

Preferably, the method includes locating the T cell antigen receptor described in the present invention close to CMV-related diseases (preferably tumors or metastatic tumors), so as to improve the efficacy of toxins or immunostimulants.

Preferably, the method is used for treating and/or preventing CMV reactivation after allogeneic hematopoietic stem cell transplantation.

Preferably, the method is used for treating and/or preventing CMV reactivation after organ transplantation (such as kidney, liver, pancreas, intestine and cornea), tissue transplantation, cell transplantation (islet cells, limbal stem cells), or stem cell therapy.

Preferably, the T cell expressing the T cell antigen receptor described in the present invention is derived from a subject.

Preferably, the T cell expressing the T cell antigen receptor described in the present invention is derived from the same donor as the hematopoietic stem cells, organs, tissues, cells or stem cells.

In the fifteenth aspect of the present invention, a method for diagnosing CMV-related diseases is provided, wherein the method includes sampling, contacting the sample with the T cell antigen receptor described in the present invention, and then detecting the complex formed by CMV pp65 and the T cell antigen receptor.

Preferably, the T cell antigen receptor includes a detectable marker.

The TCR described in the present invention can specifically recognize the corresponding CMV pp65 antigenic peptide-MHC molecular complex, activate TCR T cells, and then produce high levels of cytokines IFNy, IL2 and TNFα, all of which significantly kill tumor cells in vitro and in vivo experiments.

The "T cell antigen receptor" described in the present invention is a molecule that can recognize the peptide molecules when presented by MHC molecules or their tetramers and usually exists on the surface of T cells in the form of a complex with CD3 molecules. The TCR of most T cells is composed of α and β peptide chains, while TCR of a few T cells is composed of γ and δ peptide chains.

The "tumor" described in the present invention includes but is not limited to pancreatic cancer, liver cancer, colon cancer, rectal cancer, gastric cancer, lymphoma, basal cell cancer, non-small cell lung cancer, leukemia, ovarian cancer, nasopharyngeal cancer, breast cancer, endometrial cancer, bladder cancer, lung cancer, bronchial cancer, bone cancer, prostate cancer, cholangiocarcinoma, esophageal cancer, renal cancer, thyroid cancer, head and neck cancer, testicular cancer, glioblastoma, astrocytoma, melanoma, myelodysplastic syndrome, and sarcoma. The leukemia is selected from acute lymphocytic (lymphoblastic) leukemia, acute myeloid leukemia, myeloid leukemia, chronic lymphocytic leukemia, multiple myeloma, plasma cell leukemia and chronic myeloid leukemia. The lymphoma is selected from Hodgkin's lymphoma and non-Hodgkin's lymphoma, including B cell lymphoma, diffuse large B cell lymphoma, follicular lymphoma, mantle cell lymphoma, marginal zone B cell lymphoma, T cell lymphoma, and Waldenstrom's macroglobulinemia. The sarcoma is selected from osteosarcoma, Ewing sarcoma, leiomyosarcoma, synovial sarcoma, soft tissue sarcoma, angiosarcoma, liposarcoma, fibrosarcoma, rhabdomyosarcoma, and chondrosarcoma. Preferably, the tumor is selected from pancreatic cancer, liver cancer, oral squamous cell carcinoma, colon cancer, ovarian cancer and gastric cancer. In a specific embodiment of the present invention, the tumor is lymphoma.

The "CMV-related diseases" described in the present invention include diseases caused by CMV pp65 infection, including neonatal CMV inclusion body disease, which in severe cases can affect the liver, spleen, and central nervous system, as well as cause disability. The diseases further include acute acquired CMV infection, similar to infectious mononucleosis, including fever, skeletal muscle pain, etc. The immunocompromised individuals are further included, such as people who have transplanted organs, or people with HIV, the infection can lead to CMV retinitis, gastrointestinal CMV or encephalitis, etc.

When" comprising" or "containing" described in the present invention is adopted to describe the sequence of protein or nucleic acid, the protein or nucleic acid can be composed of the sequence, or can have additional amino acids or nucleotides at one or both ends of the protein or nucleic acid and still with the activity described in the present invention.

The "preventing" described in the present invention refers to all actions to suppress symptoms or delay the stress of specific symptoms by applying the products described in the present invention.

The "diagnosing" described in the present invention refers to finding out whether a patient has a disease or condition in the past, at the time of diagnosis or in the future, or finding out the progress of the disease or the possible progress in the future, or evaluating the patient's response to treatment.

The "treating" described in the present invention refers to slowing down, interrupting, preventing, controlling, stopping, alleviating, or reversing the progress or severity of a sign, symptom, disorder, condition, or disease, but it does not necessarily involve the complete elimination of all disease-related signs, symptoms, conditions, or disorders, and refers to therapeutic intervention to improve the signs, symptoms, etc. of a disease or pathological state after the disease has begun to develop.

An "effective amount" described in the present invention refers to the amount or dosage of the product described in the present invention that provides the desired treatment or prevention after being administered to a patient or an organ in single or multiple doses.

The "product" described in the present invention includes, but is not limited to, the antibodies or antigen-binding fragments thereof, the T cell antigen receptor, the nucleic acid, the expression vector, the host cell, the immune cell or the polymer complex, as well as other reagents that are complementary to, or synergistic with the above-mentioned products.

The "product" described in the present invention can be a pharmaceutical composition such as a kit, a chip, an antibody conjugate or a multifunctional antibody.

The "subject" described in the present invention includes, but is not limited to, a human or a non-human mammal. Preferably, the non-human mammal includes but is not limited to mice, rats, monkeys, pigs or rabbits.

The "homology" described in the present invention means that in the aspect of using amino acid sequences or nucleotide sequences, those skilled in the art can adjust the sequences as required for practical purposes without altering the primary structure or function of the original sequences, so that the used sequences, when compared to the specific sequences as described in the present invention, have (including, but not limited to) 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, or 100% homologous. For example, "having at least 80% homology to any one of the amino acid sequences as set forth in SEQ ID NO: 1-12 and SEQ ID NO: 14-73" described in the present invention means that, while retaining the binding function to the cytomegalovirus (CMV) phosphoprotein pp65 peptide epitope: MHC complex, the amino acid sequence can be adjusted as required for practical purposes, including one or more changes, such as substitution, deletion and/or insertion of one or more amino acids, etc., truncation, or extension of one or both ends, as long as it maintains more than 80% homology and retains the ability to bind to pp65 epitope/MHC complex or pp65 epitope/MHC molecule tetramer. The change could be substitution, addition or deletion. It can be the substitution between amino acids with the same polarity, the substitution between amino acids with the same charge, the substitution between uncharged amino acids, the substitution between aliphatic amino acids, the substitution between aromatic amino acids, the substitution between nonpolar amino acids, or the substitution between amino acids with functionally related side chain moieties. The basic side chain includes but is not limited to that of lysine, arginine or histidine. The acidic side chain includes but is not limited to that of aspartic acid or glutamic acid. The uncharged amino acid includes but is not limited to asparagine, glutamine, serine, threonine or tyrosine. The nonpolar side chain includes but is not limited to that of glycine, alanine, valine, leucine, isoleucine, proline, phenylalanine, methionine, tryptophan or cysteine. The "at least 80%" includes but is not limited to 80%, 81%, 82%, 83%, 84%, 85%, 86%, 87%, 88%, 89%, 90%, 91%, 92%, 93%, 94%, 95%, 96%, 97%, 98%, 99% or 100%.

Compared with the prior art, the present invention has the following beneficial effects.
1. A TCR that recognizes and binds to the CMV-pp65 antigenic epitope polypeptide fragment is obtained, which can confer transduced T cells with the ability to recognize the virus and virus-infected cells, and initiate the ability of immune responses, thus possessing specific killing capability against target cells.
2. The TCR obtained in the present invention has a novel and specific CDR3α/β structure, which is a naturally paired double-stranded structure. In addition, a single cell sequencing method is adopted in the present invention to build a database, perform multiple sequencing and comparisons of VDJ fragments of cells, so as to screen out superior sequences.
3. The nucleic acids encoding the TCR, the vectors containing the nucleic acid molecules, the cells transducing the nucleic acid molecules or the vectors, the pharmaceutical compositions containing the TCR, nucleic acid molecules, vectors or cells as active ingredients, and the uses of the TCR, nucleic acid molecules, vectors, cells and pharmaceutical compositions are further provided in the present invention, thus processing druggability and industrial production value.

### BRIEF DESCRIPTION OF THE DRAWINGS

FIG. 1 Schematic diagram of the modular structure of TCR sequence.
FIG. 2 Structure of transfer plasmid according to Example 2.
FIG. 3 Screening result of TCR-T cell transduction for positive cells according to Example 2.
FIG. 4 Screening result of TCR-T cell transduction for positive cells according to Example 5.
FIG. 5 Screening result of TCR-T cell transduction for positive cells according to Example 8.

### DETAILED DESCRIPTION OF EMBODIMENTS

Unless otherwise indicated or defined, all terms used have their ordinary meanings in the art, and will be understood by those skilled in the art. Referring to, for example, standard manuals such as Sambrook et al., "Molecular Cloning: A Laboratory Manual"; Lewin, "Genes VIII"; and Roitt et al., "Immunology" (8th edition), and the general prior art cited herein. In addition, unless otherwise specified, all methods, steps, techniques and operations not specifically detailed can be and have been carried out in a manner known per se, which will be known to those skilled in the art. Reference is also made to, for example, standard manuals, the above-mentioned general prior art and other references cited therein.

The present invention will be described below with reference to examples. The present invention is not limited to the examples.

### Example 1: Culturing, detection and sequencing of CMV-pp65₃₄₁₋₃₄₉ specific T cells

The chemically synthesized short peptide C1 (amino acid sequence: QYDPVAALF (SEQ ID NO: 13)) was adopted to stimulate peripheral blood mononuclear cells (PBMC) from healthy donors in vitro (HLA typing of healthy donors can be optional, HLA-A*2402 genotype in HLA-A*24 was taken as an example in the present example), the CD4-CD8⁺ positive CTL cells that can recognize antigenic peptide C1 and secrete IFN-γ were induced and cultivated, specifically, the following steps were included.
1) Experimental group: the X-C1 cells obtained by stimulating and culturing the donor PBMC with the antigenic peptide C1 were adopted as the experimental group; and
   Control group: without adding polypeptide to stimulate, the donor PBMC cells cultured in parallel under the same conditions as the experimental group were adopted as the control group.
2) In the detection of intracellular factor secretion, the antigenic peptide C1 was added to both groups of cells to stimulate incubation again, and whether CD8⁺IFN-γ⁺ double positive CTL cells (CD4-) recognized the antigenic peptide and secreted IFN-γ was observed; in the following experiment, the monoclonal cell strain was isolated and screened from the culture of CD4-CD8⁺ positive CTL cells which can recognize antigenic peptide and secrete IFN-γ.

### 1. Isolation and screening of monoclonal cell strains

EBV-LCL cell: Lymphoblast cell line (LCL cell line) is an immortalized cell line in vitro obtained by transfecting human PBMC with EB virus. LCL cell lines with different HLA restrictions can be prepared by using PBMC with different HLA restrictions.

The specific process of constructing immortalized human-derived B lymphocyte cell line LCLs transfected by EB virus was as follows:
1) PBMC was isolated from peripheral blood and resuspended in 2ml of RPMI1640 medium containing 10% FBS (RPMI/10% FBS for short, manufacturer of RPM1640: Thermo Fisher Scientific, item code: 22400-089; manufacturer of FBS: Thermo Fisher Scientific, item code: 10099-141C).
2) 10µL of cell suspension was pipetted, and 90µL of RPMI/10% FBS was added to achieve a 10-fold dilution, and cell counting under microscopy was performed.
3) The required volume of B95-8 supernatant (purchased from the Cell Bank of the Chinese Academy of Sciences, Catalog No. GNO₃) was calculated based on the counting results, where every 1×10⁶ PBMC cells corresponds to 500µL of B95-8 supernatant.
4) 10ml of B95-8 cells (ATCC) were cultured for two days in advance, wherein the initial density was 1×10⁶ cells/ml. After being cultured at 37°C in a 5% CO₂ incubator for 48 h, the supernatant of B95-8 cells was transferred to a centrifuge tube and centrifuged at 2000rpm for 15 min.
5) The supernatant of B95-8 cells in the centrifuge tube was filtered with a 0.45µm filter membrane for later use.
6) PBMC cells were collected. The resultant was centrifuged at 1000rpm for 5 min, and the PBMC supernatant was discarded.
7) According to the results of cell counting, a proper amount of B95-8 cell supernatant was added to resuspend PBMC cells, so as to achieve a PBMC cell concentration of 2×10⁶ cells/mL in cell suspension, then 5ml of RPMI-10 (RPMI1640 medium with 10% FBS) containing 1µg/ml of cyclosporine A was added, and mixed well, and then the above cell suspension was transferred to a 25cm² culture flask, and cultured for 3 weeks at 37°C in 5% CO₂ incubator.
8) At the end of three weeks of culture, the medium became acidic, the cells formed visible clumps, the cell volume enlarged, the cells were clear, usually hairy, and tended to form compact clumps of varying sizes, indicating that EBV induced the immortalization of B cells.

The X-C1 cells from the experimental group of CD4-CD8⁺ positive CTL cells, which can recognize antigenic peptide and secrete IFN-γ in step 1, were diluted to 0.3cell/2µl, and then the resultant was laid to a Terasaki plate (0-0.3 cells per well). EBV-LCL cells (lymphoblast cell line (LCL cell line) is an immortalized cell line in vitro obtained by transfecting human PBMC with EB virus) irradiated by 0.120J/cm² ultraviolet radiation were added as feeder cells to stimulate the growth of CTL positive T cells. After 5-7 days of static culture, the growth of monoclonal cells can be observed under the microscope. Monoclonal cell clusters were picked out and transferred to 96-well plates with U-shaped bottom, and further cultured to expansion in RPMI 1640 medium containing 30ng/ml OKT3 and 3000IU/ml IL-2. The cell culture was replenished/changed, or the culture container was changed every 3-4 days, and the irradiated EBV-LCL feeder cells were added to stimulate again every 30-40 days to maintain the specificity. In this way, the monoclonal cell strain of T cells can be obtained, which has a cell amount of 10⁷ orders of magnitude and maintains the survival rate at 80%-90%. In the amplification culture, a certain amount of cells are regularly taken for intracellular factor secretion detection to determine the existence of killing CTL cells.

### 2. Sequencing and constructing TCR.

From the culture of monoclonal cells, samples were taken for sequencing of TCR sequences, and the TCR sequence was obtained as follows.

The amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: one of CARNTGKLIF (SEQ ID NO: 3), CAPSASKIIF (SEQ ID NO: 22), and CAPQFNKFYF (SEQ ID NO: 31);
CDR1β: one of SQVTM (SEQ ID NO: 4), SGHVS (SEQ ID NO: 14), and LNHDA (SEQ ID NO: 23);
CDR2β: one of ANQGSEA (SEQ ID NO: 5), FQNEAQ (SEQ ID NO: 15), and SQIVND (SEQ ID NO: 24);
CDR3β: one of CSANPTGGGTEAFF (SEQ ID NO: 6), CASSLLTRTETQYF (SEQ ID NO: 16), and CASSTTGLAGGPGNEQFF (SEQ ID NO: 25).

The amino acid sequences of CDR1α-CDR3α of α chain and CDR1β-CDR3β of β chain of one of the TCRs obtained are shown below:
CDR1α: SSNFYA (SEQ ID NO: 1);
CDR2α: MTLNGDE (SEQ ID NO: 2);
CDR3α: CARNTGKLIF (SEQ ID NO: 3);
CDR1β: SQVTM (SEQ ID NO: 4);
CDR2β: ANQGSEA (SEQ ID NO: 5); and
CDR3β: CSANPTGGGTEAFF (SEQ ID NO: 6).

The amino acid sequences of CDR1α-CDR3α of the α chain and CDR1β-CDR3β of the β chain of another TCR obtained are shown below:
CDR1α: SSNFYA (SEQ ID NO: 1);
CDR2α: MTLNGDE (SEQ ID NO: 2);
CDR3α: CARNTGKLIF (SEQ ID NO: 3);
CDR1β: SGHVS (SEQ ID NO: 14);
CDR2β: FQNEAQ (SEQ ID NO: 15); and
CDR3β: CASSLLTRTETQYF (SEQ ID NO: 16).

The amino acid sequences of CDR1α-CDR3α of the α chain and CDR1β-CDR3β of the β chain of another TCR obtained are shown below:
CDR1α: SSNFYA (SEQ ID NO: 1);
CDR2α: MTLNGDE (SEQ ID NO: 2);
CDR3α: CAPSASKIIF (SEQ ID NO: 22);
CDR1β: LNHDA (SEQ ID NO: 23);
CDR2β: SQIVND (SEQ ID NO: 24); and
CDR3β: CASSTTGLAGGPGNEQFF (SEQ ID NO: 25).

The amino acid sequences of CDR1α-CDR3α of the α chain and CDR1β-CDR3β of the β chain of another TCR obtained are shown below:
CDR1α: SSNFYA (SEQ ID NO: 1);
CDR2α: MTLNGDE (SEQ ID NO: 2);
CDR3α: CAPQFNKFYF (SEQ ID NO: 31);
CDR1β: SGHVS (SEQ ID NO: 14);
CDR2β: FQNEAQ (SEQ ID NO: 15); and
CDR3β: CASSLLTRTETQYF (SEQ ID NO: 16).

The amino acid sequences of CDR1α-CDR3α of the α chain and CDR1β-CDR3β of the β chain of the above TCRs are shown below in Table 1:

**Table 1**

| Groups | CDR1α | CDR2α | CDR3α | CDR1β | CDR2β | CDR3β |
|---|---|---|---|---|---|---|
| First group | SSNFYA (SEQ ID NO: 1) | MTLNGDE (SEQ ID NO: 2) | CARNTGKLI F (SEQ ID NO: 3) | SQVTM (SEQ ID NO: 4) | ANQGSEA (SEQ ID NO: 5) | CSANPTGG GTEAFF (SEQ ID NO: 6) |
| Second group | SSNFYA (SEQ ID NO: 1) | MTLNGDE (SEQ ID NO: 2) | CARNTGKLI F (SEQ ID NO: 3) | SGHVS (SEQ ID NO: 14) | FQNEAQ (SEQ ID NO: 15) | CASSLLTRT ETQYF  (SEQ ID NO: 16) |
| Third group | SSNFYA (SEQ ID NO: 1) | MTLNGDE (SEQ ID NO: 2) | CAPSASKIIF (SEQ ID NO: 22) | LNHDA (SEQ ID NO: 23) | SQIVND (SEQ ID NO: 24) | CASSTTGLA GGPGNEQFF (SEQ ID NO: 25) |
| Fourth group | SSNFYA (SEQ ID NO: 1) | MTLNGDE (SEQ ID NO: 2) | CAPQFNKF YF (SEQ ID NO: 31) | SGHVS (SEQ ID NO: 14) | FQNEAQ (SEQ ID NO: 15) | CASSLLTRT ETQYF (SEQ ID NO: 16) |

On the basis of obtaining CDR1α-CDR3α of the α chain and CDR1β-CDR3β of the β chain of TCR from the first group to the fourth group in Table 1 above, the alpha sequence of TCR and the beta sequence of TCR that are paired were further obtained in the present invention, wherein the specific ways were not limited to the ways such as sequencing, and bioinformatics.

For example, the alpha sequence of TCR and the beta sequence of TCR that are paired obtained according to the first group of amino acid sequences in Table 1 are as follows:
the alpha sequence of TCR (amino acid sequence is SEQ ID NO: 9) and the beta sequence of TCR (amino acid sequence is SEQ ID NO: 10), where the alpha sequence of TCR contains variable region Vα (amino acid sequence is SEQ ID NO: 7) and the beta sequence of TCR contains variable region Vβ (amino acid sequence is SEQ ID NO: 8). The variable region Vα contains the following complementary determining regions: CDR1α: SSNFYA; CDR2α: MTLNGDE; CDR3α: CARNTGKLIF (SEQ ID NO: 1-3), and the variable region Vβ contains the following complementarity determining regions: CDR1β: SQVTM; CDR2β: ANQGSEA; CDR3β: CSANPTGGGTEAFF (SEQ ID NO: 4-6).

A leader sequence can be added upstream of the above-mentioned alpha sequence of TCR and beta sequence of TCR, respectively, and the full-length sequence of TCR (SEQ ID NO: 12) can be obtained after being connected by a linker sequence (SEQ ID NO: 11).

For example, the alpha sequence of TCR and the beta sequence of TCR that are paired obtained according to the second group of amino acid sequences in Table 1 are as follows:
the alpha sequence of TCR (amino acid sequence is SEQ ID NO: 19) and the beta sequence of TCR (amino acid sequence is SEQ ID NO: 20). The variable region Vα of alpha sequence of TCR contains the following complementarity determining regions: CDR1α: SSNFYA (SEQ ID NO: 1); CDR2α: MTLNGDE (SEQ ID NO: 2), and CDR3α: CARNTGKLIF (SEQ ID NO: 3). The variable region Vβ contains the following complementarity determining regions: CDR1β: SGHVS (SEQ ID NO: 14); CDR2β: FQNEAQ (SEQ ID NO: 15); and CDR3β: CASSLLTRTETQYF (SEQ ID NO: 16).

The above alpha sequence of TCR and beta sequence of TCR were linked by a linker sequence (SEQ ID NO: 11) to obtain the full-length sequence of TCR (SEQ ID NO: 21).

For example, the alpha sequence of TCR and the beta sequence of TCR that are paired obtained according to the third group of amino acid sequences in Table 1 are as follows:
the alpha sequence of TCR (amino acid sequence is SEQ ID NO: 28) and the beta sequence of TCR (amino acid sequence is SEQ ID NO: 29). The variable region Vα of alpha sequence of TCR contains the following complementarity determining regions: CDR1α: SSNFYA (SEQ ID NO: 1); CDR2α: MTLNGDE (SEQ ID NO: 2); and CDR3α: CAPSASKIIF (SEQ ID NO: 22). The variable region Vβ contains the following complementarity determining regions: CDR1β: LNHDA (SEQ ID NO: 23); CDR2β: SQIVND (SEQ ID NO: 24); and CDR3β: CASSTTGLAGGPGNEQFF (SEQ ID NO: 25).

The above alpha sequence of TCR and beta sequence of TCR were linked by a linker sequence (SEQ ID NO: 11) to obtain the full-length sequence of TCR (SEQ ID NO: 30).

For example, the alpha sequence of TCR and the beta sequence of TCR that are paired obtained according to the fourth group of amino acid sequences in Table 1 are as follows:
the alpha sequence of TCR (amino acid sequence is SEQ ID NO: 35) and the beta sequence of TCR (amino acid sequence is SEQ ID NO: 36). The variable region Vα of alpha sequence of TCR contains the following complementarity determining regions: CDR1α: SSNFYA (SEQ ID NO: 1); CDR2α: MTLNGDE (SEQ ID NO: 2); and CDR3α: CAPQFNKFYF (SEQ ID NO: 31). The variable region Vβ contains the following complementarity determining regions: CDR1β: SGHVS (SEQ ID NO: 14); CDR2β: FQNEAQ (SEQ ID NO: 15); and CDR3β: CASSLLTRTETQYF (SEQ ID NO: 16).

The above alpha sequence of TCR and beta sequence of TCR were linked by a linker sequence (SEQ ID NO: 11) to obtain the full-length sequence of TCR (SEQ ID NO: 36).

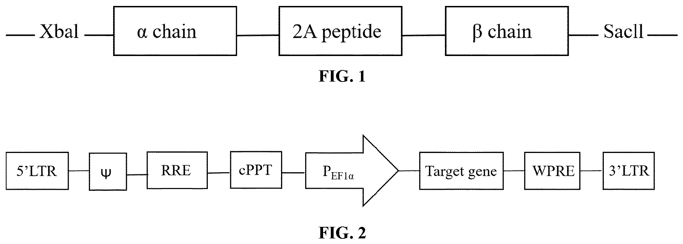

The full-length TCR containing the constant region was then constructed and inserted into the lentiviral vector. The modular structure of the TCR sequence is shown in FIG. 1.

For the convenience of explanation, the full-length sequence of TCR obtained on the basis of the first group of amino acid sequences was taken as an example in Example 2 and Example 3.

### Example 2: Preparation of CMV-pp65₃₄₁₋₃₄₉ specific TCR gene-modified T cells.

The TCR gene sequence obtained in Example 1 was cloned into a lentiviral vector and transfected into 293T viral packaging cells to prepare the viral supernatant. The viral supernatant was then transduced into T cells, so as to obtain the T cells expressing the target TCR sequence.

The transfection operation was as follows.

The lentiviral supernatant was generated by transfecting 293T cells with gag/pol packaging plasmid, VSV-G envelope plasmid and the transfer construct containing lentiviral vector sequences as described below. That is to say, the DNA mixture was mixed in Opti-MEM (Life Technologies, Gaithersburg, MD, USA) and mixed with an equal volume of Opti-MEM containing Lipofectamine 3000 (Life Technologies). After incubation at room temperature for 15 minutes, the resulting mixture was applied to 293T cells. The medium containing lentivirus was collected 24 hours post-transfection. Following each collection, the supernatant was filtered through a 0.45µm filter membrane. Lentivirus harvests were combined, stored at 4°C, and then ultracentrifuged at 20,000×g for 90 minutes. The lentiviral particles were resuspended in PBS.

Structure of transfer plasmid are shown in FIG. 2.

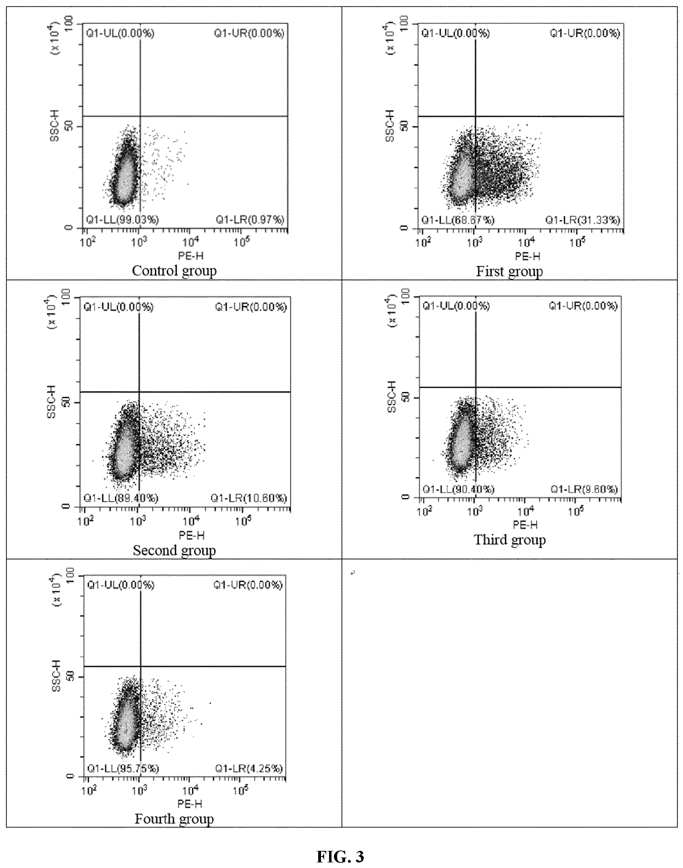

The transduction operation was as follows:
On day 0, donor blood was collected and PBMC was isolated. PBMC was activated with CD3/CD28 Dynabeads (Gibco) magnetic beads for 2 days (magnetic beads: cells = 3:1) and resuspended in serum-free medium X-VIVO15 (LONZA) containing 200 IU/ml IL-2 at 1×10⁶ cells/ml. The T cells were transduced with lentiviral supernatant on day 2, and then centrifuged at 1200×g at 32°C for 2 hours. After 24 hours, the supernatant containing the virus vector was removed. Cells were suspended in the medium containing rhIL-2 (200 IU/ml) at 3×10⁵ cells/ml and supplemented with serum-free medium X-VIVO15 with IL-2 (200 IU/ml) every 2 to 3 days. The expression level of TCR, that is, the positive rate of transduction, was detected by flow cytometry one day prior to harvest. The result is shown in FIG. 3, the non-transduced cells (NC) did not express specific TCR. The T cells transduced with lentiviral vectors containing the first group of TCR, the second of group TCR, the third group of TCR and the fourth group of TCR in Example 1 were adopted to express specific TCR (the data of CD8⁺Tetramer⁺), that is, the positive rate of transduction. The positive rate of TCR transduction in the first group was 30.36%, the positive rate of TCR transduction in the second group was 9.63%, the positive rate of TCR transduction in the third group was 8.63%, and the positive rate of TCR transduction in the fourth group was 3.28%, showing the successful transduction.

### Example 3: In vitro functional validation of T cells modified by CMV-pp65₃₄₁₋₃₄₉ specific TCR gene.

According to the present invention, by constructing target cells that overexpress the CMV-PP65 protein to which QYDPVAALF belongs, the function of TCR-T cells can be evaluated in vitro. The specific operation was as follows.
1. Preparation of effector cells: The T cells expressing the target TCR sequence prepared in Example 2 served as effector cells, wherein the T cells transfected with the TCR lentivirus and expressed were served as the TCR group, while the non-transduced T cells served as the negative control group (NC group).
2. Preparation of overexpressed target cells:

The target cell in the present invention was a tumor cell that overexpresses CMV-PP65 protein to which QYDPVAALF belongs, and was constructed by introducing a target antigen gene into the tumor cell.

Tumor cells were transduced with lentivirus containing target antigen, after 1 day, the fluid was replaced, the complete medium was used for culturing for 1-2 days, then replaced by the complete medium containing puromycin to continue the culture, and the expression of target antigen of tumor cells in transduced group was detected.

The method specifically included the following steps.

### 1) Lentiviral transduction of the target antigen gene

The complete medium (the complete medium for OS-RC-2 human renal carcinoma cells was: RPMI1640 medium (Gibco) containing 10% FBS) was adopted to adjust the density of tumor cells to 4-6×10⁵ cells/ml, 1mL per well of the resultant was added to a 6-well plate, and 8µl Polybrene (1mg/ml) was added to each well to make the final concentration was 8µg/ml. 10-30µl lentiviral vector containing the target antigen QYDPVAALF was added to each well. A cell control well (non-transduced group) was set up, wherein the control well was composed of the above 1ml tumor cells and 8µl Polybrene, which were mixed evenly and incubated in a CO₂ incubator (37°C, 5% CO₂) for 1 day.

The supernatant was discarded, 2ml of complete medium was added to each well, and the plate was incubated in a CO₂ incubator for 2 days.

### 2) Puromycin screening of transduced tumor cells

The supernatant was discarded, 2ml of complete medium containing 1µg/ml puromycin (Solarbio) was added to each well, and the plate was incubated in a CO₂ incubator.

The cells were observed every 2 days, and the complete medium containing 1µg/ml puromycin was replaced once, and the adherent cells were passaged when they were fully grown.

The complete medium containing 1µg/ml puromycin was used for screening and culturing for 7 days. If viable cells remained in the control group, it is necessary to increase the concentration of puromycin, and continue to culture until all the cells in the control group died.

### 3) Monoclonal culture of cells containing the target antigen

The tumor cells transduced with the target antigen in culture for more than 7 days were transduced and screened, and plated according to the limited dilution method with 1 cell/well and 3 cells/well. The cells were cultured in complete medium containing puromycin, and the conditions of the cells in the cell wells were observed. The monoclonal wells were marked, and continued to be cultured until the number of cells reached at least 6×10⁷ cells, then the cells were cryopreserved, thus obtaining the overexpressed target cells.

3. In vitro killing experiment: OS-RC-2-PP65 cells overexpressing PP65 protein prepared in Step 2 were plated at 5×10⁴ cells/well as target cells. The effector cells from the TCR group and NC group prepared in Step 1 were mixed with the target cells according to the effector-to-target ratio of 1:1, 5:1, 10:1 and 20:1, respectively, and co-incubated at 37°C. The killing activity was obtained using a real-time label-free cell function analyzer (xCELLigence^{®} Real Time Cell Analyzer, RTCA, Agilent).

The data at 4 hours after effector cell plating were collected to evaluate the killing activity, and the specific killing results are shown in Table 2 below.

**Table 2**

| Group | Effector-to-Target Ratio | OS-RC-2-PP65 |
|---|---|---|
| First Group of TCR | 1:1 | 83.50% |
| First Group of NC | | 0.00% |
| Second Group of TCR | | 81.60% |
| Third Group of TCR | | 84.30% |
| Fourth Group of TCR | | 62.00% |
| Second to Fourth Groups of NC | | 8.20% |
| First Group of TCR | 5:1 | 98.40% |
| First Group of NC | | 8.20% |
| Second Group of TCR | | 94.60% |
| Third Group of TCR | | 93.20% |
| Fourth Group of TCR | | 94.40% |
| Second to Fourth Groups of NC | | 58.20% |
| First Group of TCR | 10:1 | 94.30% |
| First Group of NC | | 58.20% |
| Second Group of TCR | | 91.80% |
| Third Group of TCR | | 90.80% |
| Fourth Group of TCR | | 93.50% |
| Second to Fourth Groups of NC | | 68.00% |

### Example 4: Culturing and sequencing of CMV-pp65₄₉₅₋₅₀₃ specific T cells

### 1. Antigen-specific T cell.

The chemically synthesized antigenic peptide C1 (amino acid sequence: NLVPMVATV (SEQ ID NO: 46)) was adopted to stimulate peripheral blood mononuclear cells (PBMC) from healthy donors in vitro (HLA typing of healthy donors can be optional, HLA-A*0201 genotype in HLA-A*02 was taken as an example in the present example), the CD4-CD8⁺ positive CTL cells that can recognize antigenic peptide C1 and secrete IFN-γ was induced and cultivated, specifically, the following steps were included.

3) Experimental group: the X-C1 cells cultured obtained by stimulating the donor PBMC with the antigenic peptide C1 were adopted as the experimental group; and
Control group: the donor PBMC cells cultured in parallel under the same conditions as the experimental group, without polypeptide stimulation culture, were adopted as the control group.

4) In the detection of intracellular factor secretion, the antigenic peptide C1 was added to both groups of cells to stimulate incubation again, and whether CD8⁺IFN-γ⁺ double positive CTL cells (CD4-) recognized the antigenic peptide and secreted IFN-γ was observed; in the following experiment, the monoclonal cell strain was isolated and screened from the culture of CD4-CD8⁺ positive CTL cells which can recognize antigenic peptide and secrete IFN-γ.

### 3. Isolation and screening of monoclonal cell strains

EBV-LCL cell: Lymphoblast cell line (LCL cell line) is an immortalized cell line in vitro obtained by transfecting human PBMC with EB virus. LCL cell lines with different HLA restrictions can be prepared by using PBMC with different HLA restrictions.

The specific process of constructing immortalized human-derived B lymphocyte cell line LCLs transfected by EB virus was as follows.

9) PBMC was isolated from peripheral blood and resuspended in 2ml of RPMI1640 medium containing 10% FBS (RPMI/10% FBS for short, manufacturer of RPM1640: Thermo Fisher Scientific, item code: 22400-089; manufacturer of FBS: Thermo Fisher Scientific, item code: 10099-141C).

10) 10µL of cell suspension was pipetted, and 90µL of RPMI/10% FBS was added to achieve a 10-fold dilution, and cell counting under microscopy was performed.

11) The required volume of B95-8 supernatant (purchased from the Cell Bank of the Chinese Academy of Sciences, Catalog No. GNO₃) was calculated based on the counting results, where every 1×10⁶ PBMC cells corresponds to 500µL of B95-8 supernatant.

12) 10ml of B95-8 cells (ATCC) were cultured for two days in advance, wherein the initial density was 1×10⁶ cells/ml. After being cultured at 37°C in a 5% CO₂ incubator for 48 h, the supernatant of B95-8 cells was transferred to a centrifuge tube and centrifuged at 2000rpm for 15 min.

13) The supernatant of B95-8 cells in the centrifuge tube was filtered with a 0.45µm filter membrane for later use.

14) PBMC cells were collected. The resultant was centrifuged at 1000rpm for 5 min, and the PBMC supernatant was discarded.

15) According to the results of cell counting, a proper amount of B95-8 cell supernatant was added to resuspend PBMC cells, so as to achieve a PBMC cell concentration of 2×10⁶ cells/mL in cell suspension, then 5ml of RPMI-10 (RPMI1640 medium with 10% FBS) containing 1µg/ml of cyclosporine A was added, and mixed well, and then the above cell suspension was transferred to a 25cm² culture flask, and cultured for 3 weeks at 37°C in 5% CO₂ incubator.

16) At the end of three weeks of culture, the medium became acidic, the cells formed visible clumps, the cell volume enlarged, the cells were clear, usually hairy, and tended to form compact clumps of varying sizes, indicating that EBV induced the immortalization of B cells.

The X-C1 cells from the experimental group of CD4-CD8⁺ positive CTL cells, which can recognize antigenic peptide and secrete IFN-γ in step 1, were diluted to 0.3cell/2µl, and then the resultant was laid to a Terasaki plate (0-0.3 cells per well). EBV-LCL cells (lymphoblast cell line (LCL cell line) is an immortalized cell line in vitro obtained by transfecting human PBMC with EB virus) irradiated by 0.120J/cm² ultraviolet radiation were added as feeder cells to stimulate the growth of CTL positive T cells. After 5-7 days of static culture, the growth of monoclonal cells can be observed under the microscope. Monoclonal cell clusters were picked out and transferred to 96-well plates with U-shaped bottom, and further cultured to expansion in RPMI 1640 medium containing 30ng/ml OKT3 and 3000IU/ml IL-2. The cell culture was replenished/changed, or the culture container was changed every 3-4 days, and the irradiated EBV-LCL feeder cells were added to stimulate again every 30-40 days to maintain the specificity. In this way, the monoclonal cell strain of T cells can be obtained, which has a cell amount of 10⁷ orders of magnitude and maintains the survival rate at 80%-90%. In the amplification culture, a certain amount of cells are regularly taken for intracellular factor secretion detection to determine the existence of killing CTL cells.

### 4. Sequencing and constructing TCR.

From the culture of monoclonal cells, samples were taken for sequencing of TCR sequences, and the TCR sequence was obtained as follows.

The amino acid sequences of CDR1α-CDR3α of the α chain and CDR1β-CDR3β of the β chain of the TCR are shown below:
CDR1α: one of SSNFYA (SEQ ID NO: 1), DSSSTY (SEQ ID NO: 47), and TSGFNG (SEQ ID NO: 58);
CDR2α: one of MTLNGDE (SEQ ID NO: 2), IFSNMDM (SEQ ID NO: 48), and NVLDGL (SEQ ID NO: 59);
CDR3α: one of CASINFNKFYF (SEQ ID NO: 37), CAEFTGTASKLTF (SEQ ID NO: 49), CAVTYNNARLMF (SEQ ID NO: 60), and CARNYGQNFVF (SEQ ID NO: 69);
CDR1β: one of MDHEN (SEQ ID NO: 38), GTSNPN (SEQ ID NO: 50), MNHEY (SEQ ID NO: 61), and DFQATT (SEQ ID NO: 70);
CDR2β: one of SYDVKM (SEQ ID NO: 39), SVGIG (SEQ ID NO: 51), SMNVEV (SEQ ID NO: 62), and SNEGSKA (SEQ ID NO: 71);
CDR3β: one of CASSPLNGGATEAFF (SEQ ID NO: 40), CAWSDRAAFTDTQYF (SEQ ID NO: 52), CASSSVAGGRIEQFF (SEQ ID NO: 63), and CSARDIKAQQWNIQYF (SEQ **ID** NO: 72).

In the preferred embodiment, the amino acid sequences of CDR1α-CDR3α of the α chain and CDR1β-CDR3β of the β chain of TCR are shown below:
CDR1α: SSNFYA (SEQ ID NO: 1);
CDR2α: MTLNGDE (SEQ ID NO: 2);
CDR3α: CASINFNKFYF (SEQ ID NO: 37);
CDR1β: MDHEN (SEQ ID NO: 38);
CDR2β: SYDVKM (SEQ ID NO: 39); and
CDR3β: CASSPLNGGATEAFF (SEQ ID NO: 40).

In the preferred embodiment, the amino acid sequences of CDR1α-CDR3α of the α chain and CDR1β-CDR3β of the β chain of TCR are shown below:
CDR1α: DSSSTY (SEQ ID NO: 47);
CDR2α: IFSNMDM (SEQ ID NO: 48);
CDR3α: CAEFTGTASKLTF (SEQ ID NO: 49);
CDR1β: GTSNPN (SEQ ID NO: 50);
CDR2β: SVGIG (SEQ ID NO: 51); and
CDR3β: CAWSDRAAFTDTQYF (SEQ ID NO: 52).

In the preferred embodiment, the amino acid sequences of CDR1α-CDR3α of the α chain and CDR1β-CDR3β of the β chain of TCR are shown below:
CDR1α: TSGFNG (SEQ ID NO: 58);
CDR2α: NVLDGL (SEQ ID NO: 59);
CDR3α: CAVTYNNARLMF (SEQ ID NO: 60);
CDR1β: MNHEY (SEQ ID NO: 61);
CDR2β: SMNVEV (SEQ ID NO: 62); and
CDR3β: CASSSVAGGRIEQFF (SEQ ID NO: 63).

In the preferred embodiment, the amino acid sequences of CDR1α-CDR3α of the α chain and CDR1β-CDR3β of the β chain of TCR are shown below:
CDR1α: SSNFYA (SEQ ID NO: 1);
CDR2α: MTLNGDE (SEQ ID NO: 2);
CDR3α: CARNYGQNFVF (SEQ ID NO: 69);
CDR1β: DFQATT (SEQ ID NO: 70);
CDR2β: SNEGSKA (SEQ ID NO: 71); and
CDR3β: CSARDIKAQQWNIQYF (SEQ ID NO: 72).

The amino acid sequences of CDR1α-CDR3α of the α chain and CDR1β-CDR3β of the β chain of the above TCRs are shown below in Table 3:

**Table 3**

| Groups | CDR1α | CDR2α | CDR3α | CDR1β | CDR2β | CDR3β |
|---|---|---|---|---|---|---|
| Fifth group | SSNFYA (SEQ ID NO: 1) | MTLNGDE (SEQ ID NO: 2) | CASINFNKF YF (SEQ ID NO: 37) | MDHEN (SEQ ID NO: 38) | SYDVKM (SEQ ID NO: 39) | CASSPLNGG ATEAFF (SEQ ID NO: 40) |
| Sixth group | DSSSTY (SEQ ID NO: 47) | IFSNMDM (SEQ ID NO: 48) | CAEFTGTAS KLTF (SEQ ID NO: 49) | GTSNPN (SEQ ID NO: 50) | SVGIG (SEQ ID NO: 51) | CAWSDRAA FTDTQYF (SEQ ID NO: 52) |
| Seventh group | TSGFNG (SEQ ID NO: 58) | NVLDGL (SEQ ID NO: 59) | CAVTYNNA RLMF (SEQ ID NO: 60) | MNHEY (SEQ ID NO: 61) | SMNVEV (SEQ ID NO: 62) | CASSSVAGG RIEQFF (SEQ ID NO: 63) |
| Eighth group | SSNFYA (SEQ ID NO: 1) | MTLNGDE (SEQ ID NO: 2) | CARNYGQN FVF (SEQ ID NO: 69) | DFQATT (SEQ ID NO: 70) | SNEGSKA (SEQ ID NO: 71) | CSARDIKAQ QWNIQYF (SEQ ID NO: 72) |

On the basis of obtaining CDR1α-CDR3α of the α chain and CDR1β-CDR3β of the β chain of TCR from the fifth group to the eighth group in Table 3 above, the alpha sequence of TCR and the beta sequence of TCR that are paired were further obtained in the present invention, wherein the specific ways were not limited to the ways such as sequencing, and bioinformatics.

For example, the alpha sequence of TCR and the beta sequence of TCR that are paired obtained according to the fifth group of amino acid sequences in Table 3 are as follows:
the alpha sequence of TCR (amino acid sequence is SEQ ID NO: 43) and the beta sequence of TCR (amino acid sequence is SEQ ID NO: 44), where the alpha sequence of TCR includes variable region Vα (amino acid sequence is SEQ ID NO: 41) and the beta sequence of TCR includes variable region Vβ (amino acid sequence is SEQ ID NO: 42). The variable region Vα includes the following complementary determining regions: CDR1α: SSNFYA; CDR2α: MTLNGDE; CDR3α: CASINFNKFYF (SEQ ID NO: 1, 2, 37), and the variable region Vβ includes the following complementarity determining regions: CDR1β: MDHEN; CDR2β: SYDVKM; CDR3β: CASSPLNGGATEAFF (SEQ ID NO: 38-40).

A leader sequence can be added upstream of the above-mentioned alpha sequence of TCR and beta sequence of TCR, respectively, and the full-length sequence of TCR (SEQ ID NO: 45) can be obtained after being connected by a linker sequence (SEQ ID NO: 11).

For example, the alpha sequence of TCR and the beta sequence of TCR that are paired obtained according to the sixth group of amino acid sequences in Table 3 are as follows:
the alpha sequence of TCR (amino acid sequence is SEQ ID NO: 55) and the beta sequence of TCR (amino acid sequence is SEQ ID NO: 56). The variable region Vα of alpha sequence of TCR includes the following complementarity determining regions: CDR1α: DSSSTY (SEQ ID NO: 47), CDR2α: IFSNMDM (SEQ ID NO: 48), and CDR3α: CAEFTGTASKLTF (SEQ ID NO: 49). The variable region Vβ includes the following complementarity determining regions: CDR1β: GTSNPN (SEQ ID NO: 50), CDR2β: SVGIG (SEQ ID NO: 51), and CDR3β: CAWSDRAAFTDTQYF (SEQ ID NO: 52).

The above alpha sequence of TCR and beta sequence of TCR were linked by a linker sequence (SEQ ID NO: 11) to obtain the full-length sequence of TCR (SEQ ID NO: 57).

For example, the alpha sequence of TCR and the beta sequence of TCR that are paired obtained according to the seventh group of amino acid sequences in Table 3 are as follows:
the alpha sequence of TCR (amino acid sequence is SEQ ID NO: 66) and the beta sequence of TCR (amino acid sequence is SEQ ID NO: 67). The variable region Vα of alpha sequence of TCR includes the following complementarity determining regions: CDR1α: TSGFNG (SEQ ID NO: 58), CDR2α: NVLDGL (SEQ ID NO: 59), and CDR3α: CAVTYNNARLMF (SEQ ID NO: 60). The variable region Vβ includes the following complementarity determining regions: CDR1β: MNHEY (SEQ ID NO: 61), CDR2β: SMNVEV (SEQ ID NO: 62), and CDR3β: CASSSVAGGRIEQFF (SEQ ID NO: 63).

The above alpha sequence of TCR and beta sequence of TCR were linked by a linker sequence (SEQ ID NO: 11) to obtain the full-length sequence of TCR (SEQ ID NO: 68).

For example, the alpha sequence of TCR and the beta sequence of TCR that are paired obtained according to the eighth group of amino acid sequences in Table 3 are as follows:
the alpha sequence of TCR (amino acid sequence is SEQ ID NO: 75) and the beta sequence of TCR (amino acid sequence is SEQ ID NO: 76). The variable region Vα of alpha sequence of TCR includes the following complementarity determining regions: CDR1α: SSNFYA (SEQ IDNO: 1), CDR2α: MTLNGDE (SEQ ID NO: 2), and CDR3α: CARNYGQNFVF (SEQ ID NO: 69). The variable region Vβ includes the following complementarity determining regions: CDR1β: DFQATT (SEQ ID NO: 70), CDR2β: SNEGSKA (SEQ ID NO: 71), and CDR3β: CSARDIKAQQWNIQYF (SEQ ID NO: 72).

The above alpha sequence of TCR and beta sequence of TCR were linked by a linker sequence (SEQ ID NO: 11) to obtain the full-length sequence of TCR (SEQ ID NO: 77).

The full-length TCR containing the constant region was then constructed and inserted into the lentiviral vector. The modular structure of the TCR sequence is shown in FIG. 1. For the convenience of explanation, the full-length sequence of TCR obtained on the basis of the fifth group of amino acid sequences was taken as an example in Example 5 and Example 6.

### Example 5: Preparation of CMV-pp65₄₉₅₋₅₀₃ specific TCR gene-modified T cells.

The TCR gene sequence obtained in Example 4 was cloned into a lentiviral vector and transfected into 293T viral packaging cells to prepare the viral supernatant. The viral supernatant was then transduced into T cells, so as to obtain the T cells expressing the target TCR sequence.

The transfection operation was as follows.

The lentiviral supernatant was generated by transfecting 293T cells with gag/pol packaging plasmid, VSV-G envelope plasmid and the transfer construct containing lentiviral vector sequences as described below. That is to say, the DNA mixture was mixed in Opti-MEM (Life Technologies, Gaithersburg, MD, USA) and mixed with an equal volume of Opti-MEM containing Lipofectamine 3000 (Life Technologies). After incubation at room temperature for 15 minutes, the resulting mixture was applied to 293T cells. The medium containing lentivirus was collected 24 hours post-transfection. Following each collection, the supernatant was filtered through a 0.45µm filter membrane. Lentivirus harvests were combined, stored at 4°C, and then ultracentrifuged at 20,000×g for 90 minutes. The lentiviral particles were resuspended in PBS. Structure of transfer plasmid are shown in FIG. 2.

The transduction operation was as follows.

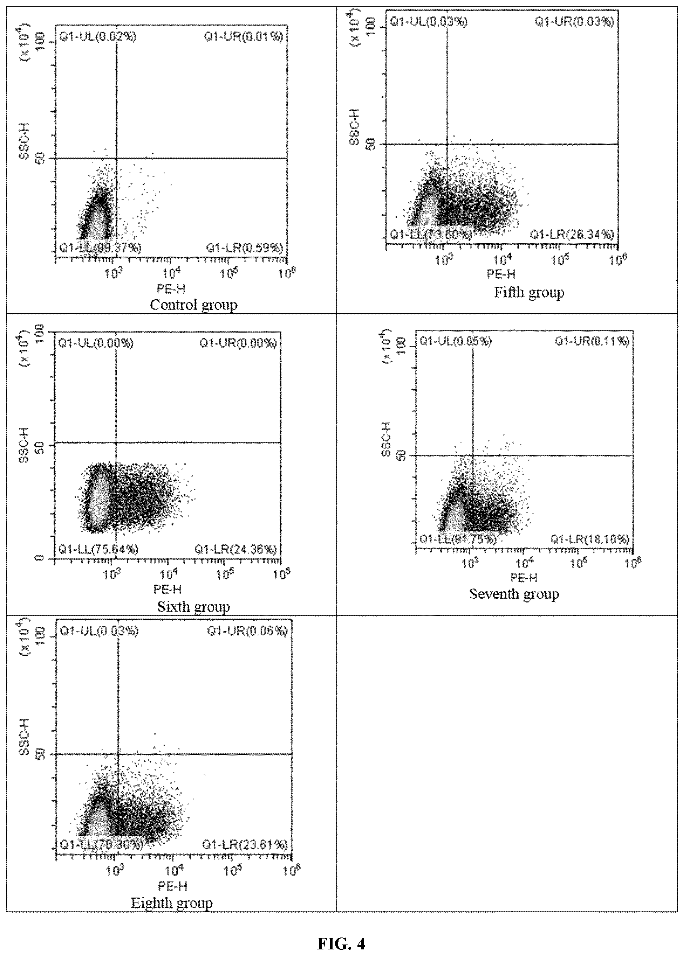

On day 0, donor blood was collected and PBMC was isolated. PBMC was activated with CD3/CD28 Dynabeads (Gibco) magnetic beads for 2 days (magnetic beads: cells = 3:1) and resuspended in serum-free medium X-VIVO15 (LONZA) containing 200 IU/ml IL-2 at 1×10⁶ cells/ml. The T cells were transduced with lentiviral supernatant on day 2, and then centrifuged at 1200×g at 32°C for 2 hours. After 24 hours, the supernatant containing the virus vector was removed. Cells were suspended in the medium containing rhIL-2 (200 IU/ml) at 3×10⁵ cells/ml and supplemented with serum-free medium X-VIVO15 with IL-2 (200 IU/ml) every 2 to 3 days. The expression level of TCR, that is, the positive rate of transduction, was detected by flow cytometry one day prior to harvest. The result is shown in FIG. 4, the non-transduced cells (NC) did not express specific TCR. The T cells transduced with lentiviral vectors containing the fifth group of TCR, the sixth of group TCR, the seventh group of TCR and the eighth group of TCR in Example 4 were adopted to express specific TCR (the data of CD8⁺Tetramer⁺), that is, the positive rate of transduction. The positive rate of TCR transduction in the fifth group was 25.75%, the positive rate of TCR transduction in the sixth group was 23.77%, the positive rate of TCR transduction in the seventh group was 17.51%, and the positive rate of TCR transduction in the eighth group was 23.02%, showing the successful transduction.

### Example 6: In vitro functional validation of T cells modified by CMV-pp65₄₉₅₋₅₀₃ specific TCR gene.

According to the present invention, by constructing target cells that overexpress the CMV-PP65 protein to which NLVPMVATV belongs, the function of TCR-T cells can be evaluated in vitro. The specific operation was as follows.
1. Preparation of effector cells: The T cells expressing the target TCR sequence prepared in Example 5 served as effector cells, wherein the T cells transfected with the TCR lentivirus and expressed were served as the TCR group, while the non-transduced T cells served as the NC group.
2. Preparation of overexpressed target cells:

The target cell in the present invention was a tumor cell that overexpresses CMV-PP65 protein to which NLVPMVATV belongs, and was constructed by introducing a target antigen gene into the tumor cell.

Tumor cells were transduced with lentivirus containing target antigen, after 1 day, the fluid was replaced, the complete medium was used for culturing for 1-2 days, then replaced by the complete medium containing puromycin to continue the culture, and the expression of target antigen of tumor cells in transduced group was detected.

The method specifically included the following steps.

### 1) Lentiviral transduction of the target antigen gene

The complete medium (the complete medium for U-2OS human osteosarcoma cells was: McCoy's 5A medium (Gibco) containing 10% FBS) was adopted to adjust the density of tumor cells to 4-6×10⁵ cells/ml, 1ml per well of the resultant was added to a 6-well plate, and 8µl Polybrene (1mg/ml) was added to each well to make the final concentration was 8µg/ml. 10-30µl lentiviral vector containing the target antigen NLVPMVATV was added to each well. A cell control well (non-transduced group) was set up, wherein the control well was composed of the above 1ml tumor cells and 8µl Polybrene, which were mixed evenly and incubated in a CO₂ incubator (37°C, 5% CO₂) for 1 day.

The supernatant was discarded, 2ml of complete medium was added to each well, and the plate was incubated in a CO₂ incubator for 2 days.

### 2) Puromycin screening of transduced tumor cells

The supernatant was discarded, 2ml of complete medium containing 1µg/ml puromycin (Solarbio) was added to each well, and the plate was incubated in a CO₂ incubator.

The cells were observed every 2 days, and the complete medium containing 1µg/ml puromycin was replaced once, and the adherent cells were passaged when they were fully grown.

The complete medium containing 1µg/ml puromycin was used for screening and culturing for 7 days. If viable cells remained in the control group, the concentration of puromycin needed to increase, and the culturing was continued until all the cells in the control group died.

### 3) Monoclonal culture of cells containing the target antigen

The tumor cells transduced with the target antigen in culture for more than 7 days were transduced and screened, and plated according to the limited dilution method with 1 cell/well and 3 cells/well. The cells were cultured in complete medium containing puromycin, and the conditions of the cells in the cell wells were observed. The monoclonal wells were marked, and continued to be cultured until the number of cells reached at least 6×10⁷ cells, then the cells were cryopreserved, thus obtaining the overexpressed target cells.

3. In vitro killing experiment: U-2OS-PP65 cells overexpressing PP65 protein prepared in Step 2 were plated at 5×10⁴ cells/well as target cells. The effector cells from the TCR group and NC group prepared in Step 1 were mixed with the target cells according to the effector-to-target ratio of 1:1, 5:1, 10:1 and 20:1, respectively, and co-incubated at 37°C. The killing activity was obtained using a real-time label-free cell function analyzer (xCELLigence^{®} Real Time Cell Analyzer, RTCA, Agilent). (The real-time label-free dynamic cell analysis technology integrates microelectronic cell sensor chips into the bottom of cell detection plates through special processes to construct a cell impedance detection sensing system that enables real-time, dynamic, and quantitative tracking of changes in cell morphology, proliferation, and differentiation.

When the cells growing adherently on the surface of the microelectrode cause changes in the impedance of the adherent electrode interface, these changes correlate with real-time alterations in cell functional states. By monitoring real-time dynamic electrode impedance, the biologically relevant information about cell physiological functions can be obtained, including cell growth, spreading, morphological changes, death, and adhesion, etc.). The data at 4 hours after effector cell plating were collected to evaluate the killing activity, and the specific killing results are shown in Table 4 below.

**Table 4**

| Group | Effector-to-Target Ratio | U-2OS-PP65 |
|---|---|---|
| Fifth Group of TCR | 1:1 | 14.3% |
| Sixth Group of TCR | | 34.6% |
| Seventh Group of TCR | | 8.6% |
| Eighth Group of TCR | | 30.2% |
| NC | | 0.0% |
| Fifth Group of TCR | 5:1 | 67.7% |
| Sixth Group of TCR | | 92.9% |
| Seventh Group of TCR | | 71.3% |
| Eighth Group of TCR | | 92.3% |
| NC | | 2.6% |
| Fifth Group of TCR | 10:1 | 88.2% |
| Sixth Group of TCR | | 93.0% |
| Seventh Group of TCR | | 82.6% |
| Eighth Group of TCR | | 93.7% |
| NC | | 37.9% |
| Fifth Group of TCR | 20:1 | 91.8% |
| Sixth Group of TCR | | 92.2% |
| Seventh Group of TCR | | 88.0% |
| Eighth Group of TCR | | 92.5% |
| NC | | 51.4% |

### Example 7: Culturing and sequencing of CMV-pp65₅₀₁₋₅₀₉ specific T cells

### 1. Antigen-specific T cell.

The chemically synthesized antigenic peptide C1 (amino acid sequence: ATVQGQNLK (SEQ ID NO: 89)) was adopted to stimulate peripheral blood mononuclear cells (PBMC) from healthy donors in vitro (HLA typing of healthy donors can be optional, HLA-A*1101 genotype in HLA-A*11 was taken as an example in the present example), the CD4-CD8⁺ positive CTL cells that can recognize antigenic peptide C1 and secrete IFN-γ was induced and cultivated, specifically, the following steps were included.

5) Experimental group: the X-C1 cells cultured obtained by stimulating the donor PBMC with the antigenic peptide C1 were adopted as the experimental group; and

Control group: the donor PBMC cells cultured in parallel under the same conditions as the experimental group, without adding polypeptide to stimulate, were adopted as the control group.

6) In the detection of intracellular factor secretion, the antigenic peptide C1 was added to both groups of cells to stimulate incubation again, and whether CD8⁺IFN-γ⁺ double positive CTL cells (CD4-) recognized the antigenic peptide and secreted IFN-γ was observed; in the following experiment, the monoclonal cell strain was isolated and screened from the culture of CD4-CD8⁺ positive CTL cells which can recognize antigenic peptide and secrete IFN-γ.

### 2. Monoclonal cell strains were isolated and screened.

EBV-LCL cell: Lymphoblast cell line (LCL cell line) is an immortalized cell line in vitro obtained by transfecting human PBMC with EB virus. LCL cell lines with different HLA restrictions can be prepared by using PBMC with different HLA restrictions.

The specific process of constructing immortalized human-derived B lymphocyte cell line LCLs transfected by EB virus was as follows.

17) PBMC was isolated from peripheral blood and resuspended in 2ml of RPMI1640 medium containing 10% FBS (RPMI/10% FBS for short, manufacturer of RPM1640: Thermo Fisher Scientific, item code: 22400-089; manufacturer of FBS: Thermo Fisher Scientific, item code: 10099-141C).

18) 10µL of cell suspension was pipetted, and 90µL of RPMI/10% FBS was added to achieve a 10-fold dilution, and cell counting under microscopy was performed.

19) The required volume of B95-8 supernatant (purchased from the Cell Bank of the Chinese Academy of Sciences, Catalog No. GNO₃) was calculated based on the counting results, where every 1×10⁶ PBMC cells corresponds to 500µL of B95-8 supernatant.

20) 10ml of B95-8 cells (ATCC) were cultured for two days in advance, wherein the initial density was 1×10⁶ cells/ml. After being cultured at 37°C in a 5% CO₂ incubator for 48 h, the supernatant of B95-8 cells was transferred to a centrifuge tube and centrifuged at 2000rpm for 15 min.

21) The supernatant of B95-8 cells in the centrifuge tube was filtered with a 0.45µm filter membrane for later use.

22) PBMC cells were collected. The resultant was centrifuged at 1000rpm for 5 min, and the PBMC supernatant was discarded.

23) According to the results of cell counting, a proper amount of B95-8 cell supernatant was added to resuspend PBMC cells, so as to achieve a PBMC cell concentration of 2×10⁶ cells/mL in cell suspension, then 5ml of RPMI-10 (RPMI1640 medium with 10% FBS) containing 1µg/ml of cyclosporine A was added, and mixed well, and then the above cell suspension was transferred to a 25cm² culture flask, and cultured for 3 weeks at 37°C in 5% CO₂ incubator.

24) At the end of the 3-week incubation, the medium became acidic, the cells formed visible clumps, the cell volume enlarged, the cells were clear, usually hairy, and tended to form compact clumps of varying sizes, indicating that EBV induced the immortalization of B cells.

The X-C1 cells from the experimental group of CD4-CD8⁺ positive CTL cells, which can recognize antigenic peptide and secrete IFN-γ in step 1, were diluted to 0.3cell/2µl, and then the resultant was laid to a Terasaki plate (0-0.3 cells per well). EBV-LCL cells (lymphoblast cell line (LCL cell line) is an immortalized cell line in vitro obtained by transfecting human PBMC with EB virus) irradiated by 0.120J/cm² ultraviolet radiation were added as feeder cells to stimulate the growth of CTL positive T cells. After 5-7 days of static culture, the growth of monoclonal cells can be observed under the microscope. Monoclonal cell clusters were picked out and transferred to 96-well plates with U-shaped bottom, and further cultured to expansion in RPMI 1640 medium containing 30ng/ml OKT3 and 3000IU/ml IL-2. The cell culture was replenished/changed, or the culture container was changed every 3-4 days, and the irradiated EBV-LCL feeder cells were added to stimulate again every 30-40 days to maintain the specificity. In this way, the monoclonal cell strain of T cells can be obtained, which has a cell amount of 10⁷ orders of magnitude and maintains the survival rate at 80%-90%. In the amplification culture, a certain amount of cells are regularly taken for intracellular factor secretion detection to determine the existence of killing CTL cells.

### 5. Sequencing and constructing TCR.

From the culture of monoclonal cells, samples were taken for sequencing of TCR sequences, and the TCR sequence was obtained as follows:

The amino acid sequences of CDR1α-CDR3α of the α chain and CDR1β-CDR3β of the β chain of the TCR are shown below:
CDR1α: one of VSGLRG (SEQ ID NO: 78), TSESDYY (SEQ ID NO: 90), and NSASQS (SEQ ID NO: 101);
CDR2α: one of LYSAGEE (SEQ ID NO: 79), QEAYKQQN (SEQ ID NO: 91), and VYSSGN (SEQ ID NO: 102);
CDR3α: one of CVITTSGTYKYIF (SEQ ID NO: 80), CAYRSFYTGANSKLTF (SEQ ID NO: 92), and CVVHSGGSYIPTF (SEQ ID NO: 103);
CDR1β: one of MNHNS (SEQ ID NO: 81), SGHDT (SEQ ID NO: 93), and MNHNY (SEQ ID NO: 104);
CDR2β: one of SASEGT (SEQ ID NO: 82), YYEEEE (SEQ ID NO: 94), and SVGAGI (SEQ ID NO: 105);
CDR3β: one of CASTINTYEQYF (SEQ ID NO: 83), CASSLYGGPGDQPQHF (SEQ ID NO: 95), and CASAQTIGAYNEQFF (SEQ ID NO: 106).

In the preferred embodiment, the amino acid sequences of CDR1α-CDR3α of the α chain and CDR1β-CDR3β of the β chain of TCR are shown below:
CDR1α: VSGLRG (SEQ ID NO: 78);
CDR2α: LYSAGEE (SEQ ID NO: 79);
CDR3α: CVITTSGTYKYIF (SEQ ID NO: 80);
CDR1β: MNHNS (SEQ ID NO: 81);
CDR2β: SASEGT (SEQ ID NO: 82); and
CDR3β: CASTINTYEQYF (SEQ ID NO: 83).

In the preferred embodiment, the amino acid sequences of CDR1α-CDR3α of the α chain and CDR1β-CDR3β of the β chain of TCR are shown below:
CDR1α: TSESDYY (SEQ ID NO: 90);
CDR2α: QEAYKQQN (SEQ ID NO: 91);
CDR3α: CAYRSFYTGANSKLTF (SEQ ID NO: 92);
CDR1β: SGHDT (SEQ ID NO: 93);
CDR2β: YYEEEE (SEQ ID NO: 94); and
CDR3β: CASSLYGGPGDQPQHF (SEQ ID NO: 95).

In the preferred embodiment, the amino acid sequences of CDR1α-CDR3α of the α chain and CDR1β-CDR3β of the β chain of TCR are shown below:
CDR1α: NSASQS (SEQ ID NO: 101);
CDR2α: VYSSGN (SEQ ID NO: 102);
CDR3α: CVVHSGGSYIPTF (SEQ ID NO: 103);
CDR1β: MNHNY (SEQ ID NO: 104);
CDR2β: SVGAGI (SEQ ID NO: 105); and
CDR3β: CASAQTIGAYNEQFF (SEQ ID NO: 106).

The amino acid sequences of CDR1α-CDR3α of the α chain and CDR1β-CDR3β of the β chain of the above TCRs are shown below in Table 5:

**Table 5**

| Groups | CDR1α | CDR2α | CDR3α | CDR1β | CDR2β | CDR3β |
|---|---|---|---|---|---|---|
| Ninth group | VSGLRG (SEQ ID NO: 78) | LYSAGEE (SEQ ID NO: 79) | CVITTSGTY KYIF (SEQ ID NO: 80) | MNHNS (SEQ ID NO: 81) | SASEGT (SEQ ID NO: 82) | CASTINTYE QYF (SEQ ID NO: 83) |
| Tenth group | TSESDYY (SEQ ID NO: 90) | QEAYKQQN (SEQ ID NO: 91) | CAYRSFYT GANSKLTF (SEQ ID NO: 92) | SGHDT (SEQ ID NO: 93) | YYEEEE (SEQ ID NO: 94) | CASSLYGG PGDQPQHF (SEQ ID NO: 95) |
| Eleventh group | NSASQS (SEQ ID NO: 101) | VYSSGN (SEQ ID NO: 102) | CVVHSGGS YIPTF (SEQ ID NO: 103) | MNHNY (SEQ ID NO: 104) | SVGAGI (SEQ ID NO: 105) | CASAQTIG AYNEQFF (SEQ ID NO: 106) |

On the basis of obtaining CDR1α-CDR3α of the α chain and CDR1β-CDR3β of the β chain of TCR from the ninth group to the eleventh group in Table 5 above, the alpha sequence of TCR and the beta sequence of TCR that are paired were further obtained in the present invention, wherein the specific ways were not limited to the ways such as sequencing, and bioinformatics.

For example, the alpha sequence of TCR and the beta sequence of TCR that are paired obtained according to the ninth group of amino acid sequences in Table 5 are as follows:
the alpha sequence of TCR (amino acid sequence is SEQ ID NO: 86) and the beta sequence of TCR (amino acid sequence is SEQ ID NO: 87), where the alpha sequence of TCR comprises variable region Vα (amino acid sequence is SEQ ID NO: 84) and the beta sequence of TCR comprises variable region Vβ (amino acid sequence is SEQ ID NO: 85). The variable region Vα comprises the following complementary determining regions: CDR1α: VSGLRG, CDR2α: LYSAGEE, and CDR3α: CVITTSGTYKYIF (SEQ ID NO: 78-80), and the variable region Vβ comprises the following complementarity determining regions: CDR1β: MNHNS; CDR2β: SASEGT; CDR3β: CASTINTYEQYF (SEQ ID NO: 81-83).

A leader sequence can be added upstream of the above-mentioned alpha sequence of TCR and beta sequence of TCR, respectively, and the full-length sequence of TCR (SEQ ID NO: 88) can be obtained after being connected by a linker sequence (SEQ ID NO: 11).

For example, the alpha sequence of TCR and the beta sequence of TCR that are paired obtained according to the tenth group of amino acid sequences in Table 5 are as follows:
the alpha sequence of TCR (amino acid sequence is SEQ ID NO: 98) and the beta sequence of TCR (amino acid sequence is SEQ ID NO: 99). The variable region Vα of alpha sequence of TCR comprises the following complementarity determining regions: CDR1α: TSESDYY, CDR2α: QEAYKQQN, and CDR3α: CAYRSFYTGANSKLTF (SEQ ID NO: 90-92). The variable region Vβ comprises the following complementarity determining regions: CDR1β: SGHDT, CDR2β: YYEEEE, and CDR3β: CASSLYGGPGDQPQHF (SEQ ID NO: 93-95).

The above alpha sequence of TCR and beta sequence of TCR were linked by a linker sequence (SEQ ID NO: 11) to obtain the full-length sequence of TCR (SEQ ID NO: 100).

For example, the alpha sequence of TCR and the beta sequence of TCR that are paired obtained according to the eleventh group of amino acid sequences in Table 5 are as follows:
the alpha sequence of TCR (amino acid sequence is SEQ ID NO: 109) and the beta sequence of TCR (amino acid sequence is SEQ ID NO: 110). The variable region Vα of alpha sequence of TCR comprises the following complementarity determining regions: CDR1α: NSASQS, CDR2α: VYSSGN, and CDR3α: CVVHSGGSYIPTF (SEQ ID NO: 101-103). The variable region Vβ comprises the following complementarity determining regions: CDR1β: MNHNY, CDR2β: SVGAGI, and CDR3β: CASAQTIGAYNEQFF (SEQ ID NO: 104-106).

The above alpha sequence of TCR and beta sequence of TCR were linked by a linker sequence (SEQ ID NO: 11) to obtain the full-length sequence of TCR (SEQ ID NO: 111).

The full-length TCR containing the constant region was then constructed and inserted into the lentiviral vector. The modular structure of the TCR sequence is shown in FIG. 1.

For the convenience of explanation, the full-length sequence of TCR obtained on the basis of the ninth group of amino acid sequences was taken as an example in Example 8 and Example 9.

### Example 8: Preparation of CMV-pp65₅₀₁₋₅₀₉ specific TCR gene-modified T cells.

The TCR gene sequence obtained in Example 7 was cloned into a lentiviral vector and transfected into 293T viral packaging cells to prepare the viral supernatant. The viral supernatant was then transduced into T cells, so as to obtain the T cells expressing the target TCR sequence.

The transfection operation was as follows.

The lentiviral supernatant was generated by transfecting 293T cells with gag/pol packaging plasmid, VSV-G envelope plasmid and the transfer construct containing lentiviral vector sequences as described below. That is to say, the DNA mixture was mixed in Opti-MEM (Life Technologies, Gaithersburg, MD, USA) and mixed with an equal volume of Opti-MEM containing Lipofectamine 3000 (Life Technologies). After incubation at room temperature for 15 minutes, the resulting mixture was applied to 293T cells. The medium containing lentivirus was collected 24 hours post-transfection. Following each collection, the supernatant was filtered through a 0.45µm filter membrane. Lentivirus harvests were combined, stored at 4°C, and then ultracentrifuged at 20,000×g for 90 minutes. The lentiviral particles were resuspended in PBS. Structure of transfer plasmid are shown in FIG. 2.

The transduction operation was as follows.

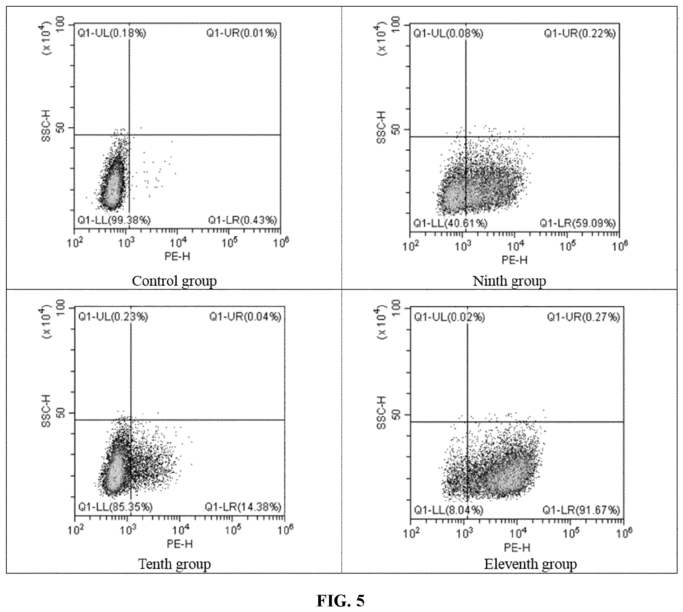

On day 0, donor blood was collected and PBMC was isolated. PBMC was activated with CD3/CD28 Dynabeads (Gibco) magnetic beads for 2 days (magnetic beads: cells = 3:1) and resuspended in serum-free medium X-VIVO15 (LONZA) containing 200 IU/ml IL-2 at 1×10⁶ cells/ml. The T cells were transduced with lentiviral supernatant on day 2, and then centrifuged at 1200×g at 32°C for 2 hours. After 24 hours, the supernatant containing the virus vector was removed. Cells were suspended in the medium containing rhIL-2 (200 IU/ml) at 3×10⁵ cells/ml and supplemented with serum-free medium X-VIVO15 with IL-2 (200 IU/ml) every 2 to 3 days. The expression level of TCR, that is, the positive rate of transduction, was detected by flow cytometry one day prior to harvest. The result is shown in FIG. 5, the non-transduced cells (NC) did not express specific TCR. The T cells transduced with lentiviral vectors containing the ninth group of TCR, the tenth of group TCR, and the eleventh group of TCR in Example 7 were adopted to express specific TCR (the data of CD8⁺Tetramer⁺), that is, the positive rate of transduction. The positive rate of TCR transduction in the ninth group was 59.09%, the positive rate of TCR transduction in the tenth group was 14.38%, and the positive rate of TCR transduction in the eleventh group was 91.67%, showing the successful transduction.

### Example 9: In vitro functional validation of T cells modified by CMV-pp65₅₀₁₋₅₀₉ specific TCR gene.

According to the present invention, by constructing target cells that overexpress the CMV-PP65 protein to which ATVQGQNLK belongs, the function of TCR-T cells can be evaluated in vitro. The specific operation was as follows.
1. Preparation of effector cells: The T cells expressing the target TCR sequence prepared in Example 8 served as effector cells, wherein the T cells transfected with the TCR lentivirus and expressed were served as the TCR group, while the non-transduced T cells served as NC group.
2. Preparation of overexpressed target cells:

The target cell in the present invention was a tumor cell that overexpresses CMV-PP65 protein to which ATVQGQNLK belongs, and was constructed by introducing a target antigen gene into the tumor cell.

Tumor cells were transduced with lentivirus containing target antigen, after 1 day, the fluid was replaced, the complete medium was used for culturing for 1-2 days, then replaced by the complete medium containing puromycin to continue the culture, and the expression of target antigen of tumor cells in transduced group was detected.

The method specifically included the following steps.

### 1) Lentiviral transduction of the target antigen gene

The complete medium (the complete medium for Caki-2 renal clear cell carcinoma cells was: McCoy's 5A medium (Gibco) containing 10% FBS) was adopted to adjust the density of tumor cells to 4-6×10⁵ cells/ml, 1ml per well of the resultant was added to a 6-well plate, and 8µl Polybrene (1mg/ml) was added to each well to make the final concentration was 8µg/ml. 10-30µl lentiviral vector containing the target antigen ATVQGQNLK was added to each well. A cell control well (non-transduced group) was set up, wherein the control well was composed of the above 1ml tumor cells and 8µl Polybrene, which were mixed evenly and incubated in a CO₂ incubator (37°C, 5% CO₂) for 1 day.

The supernatant was discarded, 2ml of complete medium was added to each well, and the plate was incubated in a CO₂ incubator for 2 days.

### 2) Puromycin screening of transduced tumor cells

The supernatant was discarded, 2ml of complete medium containing 1µg/ml puromycin (Solarbio) was added to each well, and the plate was incubated in a CO₂ incubator.

The cells were observed every 2 days, and the complete medium containing 1µg/ml puromycin was replaced once, and the adherent cells were passaged when they were fully grown.

The complete medium containing 1µg/ml puromycin was used for screening and culturing for 7 days. If viable cells remained in the control group, the concentration of puromycin needed to increase, and the culturing was continued until all the cells in the control group died.

### 3) Monoclonal culture of cells containing the target antigen

The tumor cells transduced with the target antigen in culture for more than 7 days were transduced and screened, and plated according to the limited dilution method with 1 cell/well and 3 cells/well. The cells were cultured in complete medium containing puromycin, and the conditions of the cells in the cell wells were observed. The monoclonal wells were marked, and continued to be cultured until the number of cells reached at least 6×10⁷ cells, then the cells were cryopreserved, thus obtaining the overexpressed target cells.

3. In vitro killing experiment: Caki-2-PP65 cells overexpressing PP65 protein prepared in Step 2 were plated at 5×10⁴ cells/well as target cells. The effector cells from the TCR group and NC group prepared in Step 1 were mixed with the target cells according to the effector-to-target ratio of 1:1, 5:1, and 10:1, respectively, and co-incubated at 37°C. The killing activity was obtained using a real-time label-free cell function analyzer (xCELLigence^{®} Real Time Cell Analyzer, RTCA, Agilent). (The real-time label-free dynamic cell analysis technology integrates microelectronic cell sensor chips into the bottom of cell detection plates through special processes to construct a cell impedance detection sensing system that enables real-time, dynamic, and quantitative tracking of changes in cell morphology, proliferation, and differentiation.

When the cells growing adherently on the surface of the microelectrode cause changes in the impedance of the adherent electrode interface, these changes correlate with real-time alterations in cell functional states. By monitoring real-time dynamic electrode impedance, the biologically relevant information about cell physiological functions can be obtained, including cell growth, spreading, morphological changes, death, and adhesion, etc.). The data at 4 hours after effector cell plating were collected to evaluate the killing activity, and the specific killing results are shown in Table 6 below.

**Table 6**

| Group | Effector-to-Target Ratio | Caki-2-PP65 |
|---|---|---|
| Ninth Group of TCR | 1:1 | 7.1% |
| Tenth Group of TCR | | 23.5% |
| Eleventh Group of TCR | | 1.6% |
| NC | | 0% |
| Ninth Group of TCR | 5:1 | 44.5% |
| Tenth Group of TCR | | 61.2% |
| Eleventh Group of TCR | | 50.1% |
| NC | | 0% |
| Ninth Group of TCR | 10:1 | 61.5% |
| Tenth Group of TCR | | 67.4% |
| Eleventh Group of TCR | | 66.4% |
| NC | | 0% |

The above description of specific embodiments of the present invention is not intended to limit the present invention, and those skilled in the art can make various changes or modifications according to the present invention without departing from the spirit of the present invention, which shall fall within the protection scope of the claims of the present invention.

## Claims

1. A T cell antigen receptor, **characterized in that** the T cell antigen receptor comprises a CDR1α-CDR3α of an α chain and/or a CDR1β-CDR3β of a β chain; the CDR3α comprises an amino acid sequence as set forth in one of SEQ ID NO: 3, SEQ ID NO: 22, SEQ ID NO: 31, SEQ ID NO: 37, SEQ ID NO: 49, SEQ ID NO: 60, SEQ ID NO: 69, SEQ ID NO: 80, SEQ ID NO: 92, and SEQ ID NO: 103; or comprises an amino acid sequence having at least 85% homology to one of SEQ ID NO: 3, SEQ ID NO: 22, SEQ ID NO: 31, SEQ ID NO: 37, SEQ ID NO: 49, SEQ ID NO: 60, SEQ ID NO: 69, SEQ ID NO: 80, SEQ ID NO: 92, and SEQ ID NO: 103; and/or the CDR3β comprises an amino acid sequence as set forth in one of SEQ ID NO: 6, SEQ ID NO: 16, SEQ ID NO: 25, SEQ ID NO: 40, SEQ ID NO: 52, SEQ ID NO: 63, SEQ ID NO: 72, SEQ ID NO: 83, SEQ ID NO: 95, and SEQ ID NO: 106, or comprises an amino acid sequence having at least 85% homology to one of SEQ ID NO: 6, SEQ ID NO: 16, SEQ ID NO: 25, SEQ ID NO: 40, SEQ ID NO: 52, SEQ ID NO: 63, SEQ ID NO: 72, SEQ ID NO: 83, SEQ ID NO: 95, and SEQ ID NO: 106;
preferably, the CDR1α comprises an amino acid sequence as set forth in one of SEQ ID NO: 1, SEQ ID NO: 47, SEQ ID NO: 58, SEQ ID NO: 78, SEQ ID NO: 90, and SEQ ID NO: 101, or comprises an amino acid sequence having at least 85% homology to one of SEQ ID NO: 1, SEQ ID NO: 47, SEQ ID NO: 58, SEQ ID NO: 78, SEQ ID NO: 90, and SEQ ID NO: 101; CDR2α comprises an amino acid sequence as set forth in one of SEQ ID NO: 2, SEQ ID NO: 48, SEQ ID NO: 59, SEQ ID NO: 79, SEQ ID NO: 91, and SEQ ID NO: 102, or comprises an amino acid sequence having at least 85% homology to one of SEQ ID NO: 2, SEQ ID NO: 48, SEQ ID NO: 59, SEQ ID NO: 79, SEQ ID NO: 91, and SEQ ID NO: 102; CDR1β comprises an amino acid sequence as set forth in one of SEQ ID NO: 4, SEQ ID NO: 14, SEQ ID NO: 23, SEQ ID NO: 38, SEQ ID NO: 50, SEQ ID NO: 61, SEQ ID NO: 70, SEQ ID NO: 81, SEQ ID NO: 93, and SEQ ID NO: 104; or comprises an amino acid sequence having at least 85% homology to one of SEQ ID NO: 4, SEQ ID NO: 14, SEQ ID NO: 23, SEQ ID NO: 38, SEQ ID NO: 50, SEQ ID NO: 61, SEQ ID NO: 70, SEQ ID NO: 81, SEQ ID NO: 93, and SEQ ID NO: 104; and CDR2β comprises an amino acid sequence as set forth in one of SEQ ID NO: 5, SEQ ID NO: 15, SEQ ID NO: 24, SEQ ID NO: 39, SEQ ID NO: 51, SEQ ID NO: 62, SEQ ID NO: 71, SEQ ID NO: 82, SEQ ID NO: 94, and SEQ ID NO: 105, or comprises an amino acid sequence having at least 85% homology to one of SEQ ID NO: 5, SEQ ID NO: 15, SEQ ID NO: 24, SEQ ID NO: 39, SEQ ID NO: 51, SEQ ID NO: 62, SEQ ID NO: 71, SEQ ID NO: 82, SEQ ID NO: 94, and SEQ ID NO: 105.

2. The T cell antigen receptor according to claim 1, wherein the T cell antigen receptor specific binds to CMV pp65.

3. The T cell antigen receptor according to claim 1, wherein the amino acid sequences of CDR1α-CDR3α of the α chain and the amino acid sequences of CDR1β-CDR3β of the β chain are shown below:
CDR1α: selected from one of SSNFYA (SEQ ID NO: 1), DSSSTY (SEQ ID NO: 47), TSGFNG (SEQ ID NO: 58), VSGLRG (SEQ ID NO: 78), TSESDYY (SEQ ID NO: 90), and NSASQS (SEQ ID NO: 101);
CDR2α: selected from one of MTLNGDE (SEQ ID NO: 2), IFSNMDM (SEQ ID NO: 48), NVLDGL (SEQ ID NO: 59), LYSAGEE (SEQ ID NO: 79), QEAYKQQN (SEQ ID NO: 91), and VYSSGN (SEQ ID NO: 102);
CDR3α: selected from one of CARNTGKLIF (SEQ ID NO: 3), CAPSASKIIF (SEQ ID NO: 22), CAPQFNKFYF (SEQ ID NO: 31), CASINFNKFYF (SEQ ID NO: 37), CAEFTGTASKLTF (SEQ ID NO: 49), CAVTYNNARLMF (SEQ ID NO: 60), CARNYGQNFVF (SEQ ID NO: 69), CVITTSGTYKYIF (SEQ ID NO: 80), CAYRSFYTGANSKLTF (SEQ ID NO: 92), and CVVHSGGSYIPTF (SEQ ID NO: 103);
CDR1β: selected from one of SQVTM (SEQ ID NO: 4), SGHVS (SEQ ID NO: 14), LNHDA (SEQ ID NO: 23), MDHEN (SEQ ID NO: 38), GTSNPN (SEQ ID NO: 50), MNHEY (SEQ ID NO: 61), DFQATT (SEQ ID NO: 70), MNHNS (SEQ ID NO: 81), SGHDT (SEQ ID NO: 93), and MNHNY (SEQ ID NO: 104);
CDR2β: selected from one of ANQGSEA (SEQ ID NO: 5), FQNEAQ (SEQ ID NO: 15), SQIVND (SEQ ID NO: 24), SYDVKM (SEQ ID NO: 39), SVGIG (SEQ ID NO: 51), SMNVEV (SEQ ID NO: 62), SNEGSKA (SEQ ID NO: 71), SASEGT (SEQ ID NO: 82), YYEEEE (SEQ ID NO: 94), and SVGAGI (SEQ ID NO: 105);
CDR3β: selected from one of CSANPTGGGTEAFF (SEQ ID NO: 6), CASSLLTRTETQYF (SEQ ID NO: 16), CASSTTGLAGGPGNEQFF (SEQ ID NO: 25), CASSPLNGGATEAFF (SEQ ID NO: 40), CAWSDRAAFTDTQYF (SEQ ID NO: 52), CASSSVAGGRIEQFF (SEQ ID NO: 63), CSARDIKAQQWNIQYF (SEQ ID NO: 72), CASTINTYEQYF (SEQ ID NO: 83), CASSLYGGPGDQPQHF (SEQ ID NO: 95), and CASAQTIGAYNEQFF (SEQ ID NO: 106);
preferably, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CARNTGKLIF (SEQ ID NO: 3);
CDR1β: SQVTM (SEQ ID NO: 4);
CDR2β: ANQGSEA (SEQ ID NO: 5); and
CDR3β: CSANPTGGGTEAFF (SEQ ID NO: 6);
preferably, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CARNTGKLIF (SEQ ID NO: 3);
CDR1β: SGHVS (SEQ ID NO: 14);
CDR2β: FQNEAQ (SEQ ID NO: 15); and
CDR3β: CASSLLTRTETQYF (SEQ ID NO: 16);
preferably, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CAPSASKIIF (SEQ ID NO: 22);
CDR1β: LNHDA (SEQ ID NO: 23);
CDR2β: SQIVND (SEQ ID NO: 24); and
CDR3β: CASSTTGLAGGPGNEQFF (SEQ ID NO: 25);
preferably, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CAPQFNKFYF (SEQ ID NO: 31);
CDR1β: SGHVS (SEQ ID NO: 14);
CDR2β: FQNEAQ (SEQ ID NO: 15); and
CDR3β: CASSLLTRTETQYF (SEQ ID NO: 16);
preferably, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CASINFNKFYF (SEQ ID NO: 37);
CDR1β: MDHEN (SEQ ID NO: 38);
CDR2β: SYDVKM (SEQ ID NO: 39); and
CDR3β: CASSPLNGGATEAFF (SEQ ID NO: 40);
preferably, the amino acid sequences of CDR1α-CDR3α of the α chain and CDR1β-CDR3β of the β chain of the TCR are shown below:
CDR1α: DSSSTY (SEQ ID NO: 47);
CDR2α: IFSNMDM (SEQ ID NO: 48);
CDR3α: CAEFTGTASKLTF (SEQ ID NO: 49);
CDR1β: GTSNPN (SEQ ID NO: 50);
CDR2β: SVGIG (SEQ ID NO: 51); and
CDR3β: CAWSDRAAFTDTQYF (SEQ ID NO: 52);
preferably, the amino acid sequences of CDR1α-CDR3α of the α chain and CDR1β-CDR3β of the β chain of the TCR are shown below:
CDR1α: TSGFNG (SEQ ID NO: 58);
CDR2α: NVLDGL (SEQ ID NO: 59);
CDR3α: CAVTYNNARLMF (SEQ ID NO: 60);
CDR1β: MNHEY (SEQ ID NO: 61);
CDR2β: SMNVEV (SEQ ID NO: 62); and
CDR3β: CASSSVAGGRIEQFF (SEQ ID NO: 63);
preferably, the amino acid sequences of CDR1α-CDR3α of the α chain and CDR1β-CDR3β of the β chain of the TCR are shown below:
CDR1α: SSNFYA (SEQ ID NO: 1);
CDR2α: MTLNGDE (SEQ ID NO: 2);
CDR3α: CARNYGQNFVF (SEQ ID NO: 69);
CDR1β: DFQATT (SEQ ID NO: 70);
CDR2β: SNEGSKA (SEQ ID NO: 71); and
CDR3β: CSARDIKAQQWNIQYF (SEQ ID NO: 72);
preferably, the amino acid sequences of CDR1α-CDR3α of the α chain and CDR1β-CDR3β of the β chain of the TCR are shown below:
CDR1α: VSGLRG (SEQ ID NO: 78);
CDR2α: LYSAGEE (SEQ ID NO: 79);
CDR3α: CVITTSGTYKYIF (SEQ ID NO: 80);
CDR1β: MNHNS (SEQ ID NO: 81);
CDR2β: SASEGT (SEQ ID NO: 82); and
CDR3β: CASTINTYEQYF (SEQ ID NO: 83);
preferably, the amino acid sequences of CDR1α-CDR3α of the α chain and CDR1β-CDR3β of the β chain of the TCR are shown below:
CDRlα: TSESDYY (SEQ ID NO: 90);
CDR2α: QEAYKQQN (SEQ ID NO: 91);
CDR3α: CAYRSFYTGANSKLTF (SEQ ID NO: 92);
CDRlβ: SGHDT (SEQ ID NO: 93);
CDR2β: YYEEEE (SEQ ID NO: 94); and
CDR3β: CASSLYGGPGDQPQHF (SEQ ID NO: 95);
preferably, the amino acid sequences of CDR1α-CDR3α of the α chain and CDR1β-CDR3β of the β chain of the TCR are shown below:
CDRlα: NSASQS (SEQ ID NO: 101);
CDR2α: VYSSGN (SEQ ID NO: 102);
CDR3α: CVVHSGGSYIPTF (SEQ ID NO: 103);
CDR1β: MNHNY (SEQ ID NO: 104);
CDR2β: SVGAGI (SEQ ID NO: 105); and
CDR3β: CASAQTIGAYNEQFF (SEQ ID NO: 106).

4. The T cell antigen receptor according to any one of claims 1-3, wherein an α chain variable region comprises an amino acid sequence as set forth in SEQ ID NO: 7, SEQ ID NO: 17, SEQ ID NO: 26, SEQ ID NO: 32, SEQ ID NO: 41, SEQ ID NO: 53, SEQ ID NO: 64, SEQ ID NO: 73, SEQ ID NO: 84, SEQ ID NO: 96, or SEQ ID NO: 107; or comprises an amino acid sequence having at least 85% homology to SEQ ID NO: 7, SEQ ID NO: 17, SEQ ID NO: 26, SEQ ID NO: 32, SEQ ID NO: 41, SEQ ID NO: 53, SEQ ID NO: 64, SEQ ID NO: 73, SEQ ID NO: 84, SEQ ID NO: 96, or SEQ ID NO: 107;
preferably, a β chain variable region comprises the amino acid sequence as set forth in SEQ ID NO: 8, SEQ ID NO: 18, SEQ ID NO: 27, SEQ ID NO: 33, SEQ ID NO: 42, SEQ ID NO: 54, SEQ ID NO: 65, SEQ ID NO: 74, SEQ ID NO: 85, SEQ ID NO: 97, or SEQ ID NO: 108; or comprises an amino acid sequence having at least 85% homology to SEQ ID NO: 8, SEQ ID NO: 18, SEQ ID NO: 27, SEQ ID NO: 33, SEQ ID NO: 42, SEQ ID NO: 54, SEQ ID NO: 65, SEQ ID NO: 74, SEQ ID NO: 85, SEQ ID NO: 97, or SEQ ID NO: 108;
preferably, the α chain comprises an amino acid sequence as set forth in SEQ ID NO: 9, SEQ ID NO: 19, SEQ ID NO: 28, SEQ ID NO: 34, SEQ ID NO: 43, SEQ ID NO: 55, SEQ ID NO: 66, SEQ ID NO: 75, SEQ ID NO: 86, SEQ ID NO: 98, or SEQ ID NO: 109; or comprises an amino acid sequence having at least 85% homology to SEQ ID NO: 9, SEQ ID NO: 19, SEQ ID NO: 28, SEQ ID NO: 34, SEQ ID NO: 43, SEQ ID NO: 55, SEQ ID NO: 66, SEQ ID NO: 75, SEQ ID NO: 86, SEQ ID NO: 98, or SEQ ID NO: 109; and
preferably, the β chain comprises an amino acid sequence as set forth in SEQ ID NO: 10, SEQ ID NO: 20, SEQ ID NO: 29, SEQ ID NO: 35, SEQ ID NO: 44, SEQ ID NO: 56, SEQ ID NO: 67, SEQ ID NO: 76, SEQ ID NO: 87, SEQ ID NO: 99, or SEQ ID NO: 110; or comprises an amino acid sequence having at least 85% homology to SEQ ID NO: 10, SEQ ID NO: 20, SEQ ID NO: 29, SEQ ID NO: 35, SEQ ID NO: 44, SEQ ID NO: 56, SEQ ID NO: 67, SEQ ID NO: 76, SEQ ID NO: 87, SEQ ID NO: 99, or SEQ ID NO: 110.

5. The T cell antigen receptor according to any one of claims 1-4, wherein amino acids of the α chain and the β chain are directly or indirectly connected, preferably indirectly connected, and more preferably connected by using a fp2A; preferably, an amino acid sequence of the fp2A is as set forth in SEQ ID NO: 11;
preferably, the T cell antigen receptor comprises an amino acid sequence as set forth in SEQ ID NO: 12, SEQ ID NO: 21, SEQ ID NO: 30, SEQ ID NO: 36, SEQ ID NO: 45, SEQ ID NO: 57, SEQ ID NO: 68, SEQ ID NO: 77, SEQ ID NO: 88, SEQ ID NO: 100, or SEQ ID NO: 111; or comprises an amino acid sequence having at least 85% or homology to SEQ ID NO: 12, SEQ ID NO: 21, SEQ ID NO: 30, SEQ ID NO: 36, SEQ ID NO: 45, SEQ ID NO: 57, SEQ ID NO: 68, SEQ ID NO: 77, SEQ ID NO: 88, SEQ ID NO: 100, or SEQ ID NO: 111.

6. A nucleic acid, **characterized in that** the nucleic acid encodes the T cell antigen receptor according to any one of claims 1-5.

7. An expression vector, **characterized in that** the expression vector comprises the nucleic acid according to claim 6.

8. A host cell, **characterized in that** the host cell comprises the nucleic acid according to claim 6 or the expression vector according to claim 7.

9. An immune cell, **characterized in that** the immune cell expresses the T cell antigen receptor according to any one of claims 1-5.

10. A preparation method for immune cells, **characterized in that** the preparation method includes transducing a nucleic acid sequence encoding the nucleic acid according to claim 6 into the immune cells for expression.

11. A preparation method for recombinant T cells, **characterized in that** the method includes following steps:
1) obtaining the nucleic acid according to claim 6 by cloning from CMV positive T cells;
2) isolating and culturing a primary T cell; and
3) delivering the nucleic acid obtained in step 1) to the primary T cell described in step 2), so as to obtain the recombinant T cells expressing the T cell antigen receptor according to any one of claims 1-5.

12. A preparation method for a T cell antigen receptor, **characterized in that** the method includes following steps:
(1) obtaining the nucleic acid according to claim 6 by cloning from positive T cells;
(2) linking the nucleic acid obtained in step (1) to a vector to obtain an expression vector;
(3) transforming the expression vector obtained in step (2) into a host cell, and then inducing its expression; and
(4) obtaining the T cell antigen receptor.

13. Use of the T cell antigen receptor according to any one of claims 1-5, the nucleic acid according to claim 6, the expression vector according to claim 7, the host cell according to claim 8, and the immune cell according to claim 9, in preparing a product for diagnosing, preventing or treating CMV-related diseases, or in T cell labeling, detection, cell sorting or activation.

14. A pharmaceutical composition, **characterized in that** the pharmaceutical composition comprises any one of following groups:
1) the T cell antigen receptor according to any one of claims 1-5;
2) the nucleic acid according to claim 6;
3) the expression vector according to claim 7;
4) the host cell according to claim 8; or
5) the immune cell according to claim 9.

15. A kit, **characterized in that** the kit comprises any one of following groups:
1) the T cell antigen receptor according to any one of claims 1-5;
2) the nucleic acid according to claim 6;
3) the expression vector according to claim 7;
4) the host cell according to claim 8;
5) the immune cell according to claim 9.
